# EUROPÄISCHE PATENTSCHRIFT

(11) **EP 1 718 347 B1**
(45) Veröffentlichungstag und Bekanntmachung des Hinweises auf die Patenterteilung: **08.03.2017**
(21) Anmeldenummer: 05715031.0
(22) Anmeldetag: 27.02.2005
(51) Int. Cl.: A61L 31/10, A61L 31/14, A61L 31/16

(54) **BIOKOMPATIBLE BESCHICHTUNG, VERFAHREN UND VERWENDUNG VON MEDIZINISCHEN OBERFLÄCHEN**
BIOCOMPATIBLE COATING, METHOD, AND USE OF MEDICAL SURFACES
REVÊTEMENT BIOCOMPATIBLE, PROCÉDÉ ET UTILISATION DE SURFACES MÉDICALES

(30) Priorität: 28.02.2004 DE 102004009850; 11.03.2004 US 551761 P
(43) Veröffentlichungstag der Anmeldung: 08.11.2006
(73) Patentinhaber: Hemoteq AG, 52146 Würselen (DE)
(72) Erfinder: HOFFMANN, Erika, 52249 Eschweiler (DE)
(74) Vertreter: Arth, Hans-Lothar
(86) Internationale Anmeldenummer: PCT/DE2005/000327
(87) Internationale Veröffentlichungsnummer: WO 2005/082434

(56) Entgegenhaltungen:
- EP-A- 0 404 683
- EP-A- 0 593 284
- EP-A- 0 610 086
- EP-A- 0 790 823
- EP-A- 0 839 541
- WO-A-01/01957
- WO-A-02/100559
- WO-A-03/039612
- US-A1- 2003 125 800

## Beschreibung

In den letzten Jahren hat das Einsetzen von Stents bei der Ballondilatation von verschlossenen Blutgefäßen immer weitere Verbreitung gefunden. Obwohl Stents das Risiko eines erneuten Gefäßverschlusses verkleinern, sind sie bisher nicht in der Lage, diese Restenosen vollständig zu verhindern.

Eine genaue begriffliche Beschreibung der Restenose ist in der Fachliteratur nicht aufzufinden. Die am häufigsten verwendete Definition der Restenose ist morphologisch begründet und legt diesen Begriff als eine Reduktion des Gefäßdurchmessers auf weniger als 50% des normalen nach erfolgreicher PTA (perkutane transluminale Angioplastie) fest. Somit handelt es sich um einen empirisch festgelegten Wert, dessen hämodynamische Bedeutung und Beziehung zur klinischen Symptomatik ohne solide wissenschaftliche Basis ist. In der Praxis wird häufig die klinische Verschlechterung eines Patienten als Zeichen einer Restenose des vormals behandelten Gefäßabschnitts angesehen.

Für die durch den Stent verursachte Restenose gibt es zwei verschiedene Ursachen:
a.) In der ersten Zeit nach der Implantation ist die Oberfläche des Stent dem Blut direkt ausgesetzt und es kann aufgrund der nun vorhandenen Fremdoberfläche zu einer akuten Thrombose kommen, die das Blutgefäß wieder verschließt.
b.) Mit Implantation des Stent werden Gefäßverletzungen verursacht, die neben der oben genannten Thrombose, ebenfalls Entzündungsreaktionen hervorrufen, die für den Heilungsprozeß in den ersten sieben Tagen eine entscheidende Rolle spielen. Die hierbei ablaufenden Prozesse sind unter anderem mit der Ausschüttung von Wachstumsfaktoren verbunden, womit eine verstärkte Proliferation der glatten Muskelzellen eingeleitet wird und damit schon kurzfristig zu einem erneuten Verschluß des Gefäßes aufgrund unkontrollierten Wachstums führen.
c.) Nach einigen Wochen beginnt der Stent in das Gewebe des Blutgefäßes einzuwachsen. Das heißt, daß der Stent vollständig von glatten Muskelzellen umhüllt wird und keinen Kontakt mehr zum Blut hat. Diese Vernarbung kann zu stark ausgeprägt sein (Neointimahyperplasie) und dazu führen, daß nicht nur die Stentoberfläche bedeckt, sondern der ganze Innenraum des Stent verschlossen wird.

Man hat vergeblich versucht, das Problem der Restenose durch die Beschichtung der Stents mit Heparin zu lösen (J. Whörle et al, European Heart Journal 2001, 22, 1808-1816). Heparin adressiert als Antikoagulanz jedoch nur die erstgenannte Ursache und kann darüber hinaus nur in Lösung seine volle Wirkung entfalten. Dieses erste Problem läßt sich mittlerweile medikamentös durch die Gabe von Antikoagulantien fast vollständig vermeiden. Das zweite und dritte Problem versucht man zur Zeit zu lösen, indem man das Wachstum der glatten Muskelzellen lokal am Stent hemmt. Das wird z.B. mit radioaktiven Stents oder mit Stents versucht, deren Oberfläche mit biokompatiblen Materialien bedeckt ist als auch mit Stents, die pharmazeutische Wirkstoffe abgeben.

So offenbart US-A-5 891 108 beispielsweise einen hohl ausgeformten Stent, welcher in seinem Inneren pharmazeutische Wirkstoffe enthalten kann, welche durch eine Vielzahl von Öffnungen im Stent freigesetzt werden. EP-A-1 127 582 beschreibt hingegen einen Stent, der auf seiner Oberfläche Einbuchtungen von 0,1 - 1 mm Tiefe und 7 - 15 mm Länge aufweist, welche zur Aufnahme eines Wirkstoffes geeignet sind. Diese Wirkstoffreservoire setzen, vergleichbar der Öffnungen in dem hohlen Stent, den enthaltenen pharmazeutischen Wirkstoff punktuell in hoher Konzentration und über einen relativ langen Zeitraum frei, was aber dazu führt, daß die glatten Muskelzellen nicht mehr oder nur sehr verzögert in der Lage sind, den Stent zu umhüllen. Daher ist der Stent viel länger dem Blut ausgesetzt, was wieder vermehrt zu Gefäßverschlüssen durch Thrombosen führt (Liistro F., Colombo A., Late acute thrombosis after Paclitaxel eluting stent implantation. Heart 2001, 86, 262-4).

Ein Lösungsversuch für dieses Problem stellt die Phosphorylcholinbeschichtung von biokompatiblen Oberflächen (WO 0101957) dar, indem hier Phosphorylcholin, eine Zellmembrankomponente der Erythrocyten, als Bestandteil der aufgebrachten nicht bioabbaubaren Polymerschicht auf dem Stent eine nichtthrombogene Oberfläche erzeugen soll. Dabei wird der Wirkstoff abhängig vom Molekulargewicht von dieser polymerhaltigen Phosphorylcholinschicht absorbiert oder auf der Oberfläche adsorbiert.

Phosphorylcholin gehört zur Gruppe der membranbildenden Phosphoglyceride, die aus einem Glycerinmolekül bestehen, an dessen ersten und zweiten Hydroxylgruppe vor allem längerkettige gesättigte und ungesättigte Fettsäuren wie die Palmitinsäure (C16), die Stearinsäure (C18) und die Ölsäure (C18:1) verestert sind, während die dritte Hydroxylgruppe Phosphorsäure bindet. Die Phosphorsäure bildet mit einem zweiten Alkohol, z.B. Cholin, ebenfalls einen Ester, der als das polare Kopfteil bezeichnet wird.

Fettsäuren sind wasserunlösliche, ölige oder fettige Substanzen die neben Wasser, Enzymen und Kohlenhydraten wichtige Biomoleküle darstellen, die entweder in Form der Triacylglycerine als Brennstoff zur Gewinnung chemischer Energie dienen und gespeichert werden können oder in Form von membranbildenden Verbindungen wie z.B. die schon erwähnten Phosphoglyceride und Sphingolipide die Entstehung und den Bestand der Zelle sichern.

EP 0 790 823 nutzt diese Lipide beispielsweise zur Darstellung von wirkstoffeinschließenden Liposomen, die in einem polymeren Drug delivery Material dazu beitragen, den Wirkstoff an der damit beschichteten medizinischen Oberfläche zu halten.

Die Herstellung der Triacylglycerine und der Phospholipide stellt einen äußerst aktiven Stoffwechselprozeß dar, der in jeder Zelle stattfindet. Die beiden synthetisierten Fettsäuren Palmitinsäure (C16) und Stearinsäure (C18) sind ebenfalls die Vorstufen für die weit verbreiteten einfach ungesättigten Fettsäuren in tierischen Geweben, wie die Palmitoleinsäure (C16:1) und die Ölsäure (C18:1).

Alle weiteren wichtigen ungesättigten Fettsäuren sind als essentielle Fettsäuren über die Nahrung aufzunehmen. Unter anderem ist hier die Linolsäure, eine Omega-6-Fettsäure, zu erwähnen, die letztendlich vom Organismus in Arachidonsäure (C20) umgewandelt wird, die von entscheidender Bedeutung als Vorstufe zur Synthese von Thromboxanen und Prostaglandinen dienen, die wiederum viele verschiedene wichtige Zellfunktionen regulieren.

EP 0 404 683 B1 beschreibt den Einsatz von Fettsäuren auf medizinischen Oberflächen, die mit Blut in Kontakt stehen. Die Fettsäuren, und im besonderen die Linolensäure, werden zur Verbesserung der Hämokompatibilität des eingesetzten hydrophilen Polymeren an dieses kovalent gebunden. Erwähnte Anwendungsbeispiele sind künstliche Organe, Dialysatoren, Blutfilter und Katheter. Doch der Aufwand der Herstellung dieses Beschichtungssystems ist hoch und die benötigten Kopplungssubstanzen nicht ungefährlich, so dass eine solche Beschichtung nach unserer Kenntnis bisher nicht vermarktet wird. Zudem werden die Fettsäuren über einen Spacer an das Polymer gebunden, wobei die Fettsäuren über eine Amidbindung an den Spacer gebunden werden.

WO-03039612 bezieht sich gleichfalls auf die bekannte antithrombotische und antiproliferative Wirkung der ungesättigten Fettsäuren auf das cardiovasculäre System und beschreibt erstmals eine Beschichtung von Stents mit käuflichen Ölen wie Olivenöl, Sonnenblumenöl, Palmöl und Fischöl und besonders von Lebertran. Die verwendeten flüssigen Öle werden als antithrombotische Beschichtung eingesetzt, wobei auch Emulsionen unter Zusatz von Wirkstoffen aufgetragen werden. Doch gibt es hier zu bedenken, dass die gleichmäßige Verteilung des flüssigen Öls auf einem Stent sich sicher sehr schwierig gestaltet und der Stent zum nicht unerheblichen Teil unbeschichtet bleibt. Der Stent verliert zudem auf dem Wege zum Bestimmungsort weitere Beschichtungssubstanz, womit die unbeschichteten Flächen noch größer werden und der Wirkstoffgehalt, der tatsächlich am Ziel zur Verfügung steht, letztendlich nur äußerst schwer zu ermitteln ist.

Zudem ist die Haltbarkeit der Beschichtung und damit auch die zur Verfügung stehende Zeit des zugesetzten Wirkstoffes durch die Beschichtung selbst stark eingegrenzt, da sich die Matrix nach einiger Zeit auflöst, womit der Restenoserate des eingesetzten unbeschichteten Stents wieder eine wesentliche Rolle zukommt.

Aufgabe der vorliegenden Erfindung ist es, hämokompatible Oberflächen von Stents bereitzustellen. Vorzugsweise sind derartige Oberflächen zudem in der Lage, einen oder mehrere antiproliferative, antiinflammatorische, antiangiogene und/oder antithrombotische Wirkstoffe kontrolliert freizusetzen. Insbesondere ist es Aufgabe der vorliegenden Erfindung, Stents bereitzustellen, welche ein kontinuierliches kontrolliertes Einwachsen des Stent in die Gefäßwand durch die Bereitstellung einer biokompatiblen Oberfläche als Matrix gewährleisten und durch ihren Abbau keine Reaktionen auf die bestehende Fremdoberfläche mehr verursachen, was sonst langzeitlich zu einem Wiederverschluß des Blutgefäßes führen könnte.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

Es hat sich überraschend herausgestellt, dass man Substanzen, die mindestens einen linearen oder verzweigten und einen substituierten oder unsubstituierten Alkylrest mit mindestens einer Mehrfachbindung enthalten, nach der Auftragung auf die Oberfläche eines Stents an der Luft zu einem Harz polymerisieren kann, in welches beispielsweise auch noch pharmazeutische Wirkstoffe eingeschlossen werden können und wobei durch die Polymerisation eine biokompatible Beschichtung der medizinischen Oberfläche erreicht wird, wobei die an der Polymerisationsreaktion teilnehmenden Substanzen einfach oder mehrfach ungesättigte Fettsäuren und/oder Mischungen aus diesen ungesättigten Fettsäuren in Form ihrer Triglyceride und/oder in nicht glyceringebundener, freier Form sind.

Diese an der Polymerisationsreaktion aktiv teilnehmenden Substanzen, die mindestens einen linearen oder verzweigten und einen substituierten oder unsubstituierten Alkylrest mit mindestens einer Mehrfachbindung tragen, sind Substanzen mit mindestens einem ungesättigten Fettsäurerest.

Zu den Substanzen, welche mindestens einen Alkylrest mit mindestens einer Mehrfachbindung, d.h. einen ungesättigten Fettsäurerest enthalten, gehören beispielsweise Fettsäuren, deren Triglyceride, sowie Mischungen der vorgenannten Substanzen.

Erfindungsgemäß werden diese an der Polymerisationsreaktion teilnehmenden Substanzen, welche mindestens einen Alkylrest oder Fettsäurerest mit mindestens einer Mehrfachbindung enthalten, durch Einwirkung von Wärme, von Licht und/oder von Luftsauerstoff miteinander über diese mindestens eine Mehrfachbindung polymerisiert. Bei dieser Polymerisation kann ein Katalysator in einer biologisch bzw. pharmakologisch verträglichen Konzentration eingesetzt werden. Insbesondere vorteilhaft ist es, wenn die mindestens einen Alkylrest mit mindestens einer Mehrfachbindung enthaltende Substanzen zur Autopolymerisation befähigt sind.

In diese bei der Polymerisation entstehenden Matrix können diverse weitere Substanzen, welche nicht aktiv an der Polymerisation teilnehmen, sondern in der gebildeten Polymermatrix eingeschlossen werden, eingebracht werden. Diese werden weiter unten beschrieben.

Ferner können die vorgenannten Verbindungen im Gemisch mit nicht an der Polymerisation teilnehmenden Substanzen und insbesondere im Gemisch mit den hierin genannten Ölen und/oder Fettsäuren eingesetzt werden. Bevorzugt sind derartige Gemische und Einzelsubstanzen, welche zur Polymerisation, insbesondere zur Autopolymerisation geeignet sind.

Die an der Polymerisation teilnehmenden Substanzen umfassen einfach oder mehrfach ungesättigte Fettsäuren und/oder Mischungen aus diesen ungesättigten Fettsäuren in Form ihrer Triglyceride und/oder in nicht glyceringebundener, freier Form.

Vorzugsweise werden die ungesättigten Fettsäuren aus der Gruppe ausgewählt, welche Ölsäure, Eicosapentaensäure, Timnodonsäure, Docosahexaensäure, Arachidonsäure, Linolsäure, α-Linolensäure, γ-Linolensäure sowie Mischungen der vorgenannten Fettsäuren umfasst. Diese Mischungen umfassen insbesondere Mischungen der reinen ungesättigten Verbindungen.

Fischöl und Lebertran enthalten hauptsächlich Eicosapentaensäure (EPA C20:5) und Docosahexaensäure (DHA C22:6) neben wenig alpha-Linolensäure (ALA C18:3). Bei allen drei Fettsäuren handelt es sich um Omega-3-Fettsäuren, die im Organismus als bedeutende biochemische Bausubstanz für zahlreiche Zellstrukturen benötigt werden (DHA und EPA), zum Beispiel sind sie wie bereits erwähnt, wesentlich für den Aufbau und Bestand der Zellmembran (Sphingolipide, Ceramide, Ganglioside) verantwortlich.

Omega-3-Fettsäuren finden sich nicht nur in Fischöl, sondern auch in Pflanzenölen. Weitere ungesättigte Fettsäuren, wie die Omega-6-Fettsäuren, sind in Ölen pflanzlicher Herkunft vorhanden, die hier teils einen höheren Anteil als in tierischen Fetten ausmachen. Daher werden verschiedene Pflanzenöle wie Leinöl, Walnussöl, Flachsöl, Nachtkerzenöl mit entsprechend hohem Gehalt an essentiellen Fettsäuren als besonders hochwertige und wertvolle Speiseöle empfohlen. Vor allem Leinöl stellt ein wertvoller Lieferant von Omega-3- und Omega-6-Fettsäuren dar und ist als hochwertiges Speiseöl seit Jahrzehnten bekannt.

Als an der Polymerisationsreaktion teilnehmende Substanzen sind die Omega-3 als auch die Omega-6-Fettsäuren bevorzugt sowie sämtliche Substanzen, welche mindestens einen Omega-3 und/oder Omega-6-Fettsäurerest tragen. Derartige Substanzen zeigen auch eine gute Befähigung zur Autopolymerisation.

Die Fähigkeit auszuhärten, d.h. die Fähigkeit zur Autopolymerisation, ist in der Zusammensetzung der auch als trocknende Öle bezeichneten Öle begründet und geht auf den hohen Gehalt an essentiellen Fettsäuren, genauer auf die Doppelbindungen der ungesättigten Fettsäuren zurück. An der Luft werden mit Hilfe des Sauerstoffs an den Doppelbindungsstellen der Fettsäuremoleküle Radikale gebildet, die die radikalische Polymerisation initiieren und propagieren, so dass die Fettsäuren unter Verlust der Doppelbindungen untereinander vernetzen. Durch die Aufhebung der Doppelbindung im Fettmolekül steigt der Schmelzpunkt und die Vernetzung der Fettsäuremoleküle bewirkt eine zusätzliche Härtung. Es entsteht ein hochmolekulares Harz, dass als flexibler Polymerfilm die medizinische Oberfläche gleichmäßig bedeckt.

Die Autopolymerisation wird auch als Selbstpolymerisation bezeichnet und kann beispielsweise durch Sauerstoff, insbesondere Luftsauerstoff initiiert werden. Diese Autopolymerisation kann auch unter Lichtausschluss durchgeführt werden. Eine weitere Möglichkeit besteht in der Initiierung der Autopolymerisation durch elektromagnetische Strahlung, insbesondere Licht. Eine weitere aber weniger bevorzugt Variante stellt die Autopolymerisation ausgelöst durch chemische Zerfallsreaktionen dar, insbesondere Zerfallsreaktionen der zu polymerisierenden Stoffe.

Je mehr Mehrfachbindungen der Fettsäurerest aufweist, desto ist der Vernetzungsgrad. Somit ist die Menge an Substanzen, welche aktiv an der Polymerisationsreaktion teilnehmen um so geringer, je höher die Dichte an Mehrfachbindungen in einem Alkylrest (Fettsäurerest) als auch in einem Molekül ist.

Der Gehalt an aktiv an der Polymerisationsreaktion teilnehmenden Substanzen in Bezug auf die Gesamtmenge aller auf die Oberfläche des Medizinproduktes aufgetragenen Substanzen beträgt mindestens 25 Gew.-%, bevorzugt 35 Gew.-%, weiter bevorzugt 45 Gew.-% und insbesondere bevorzugt 55 Gew.-%.

Die folgende Tabelle 1 zeigt eine Aufstellung der Fettsäurebestandteile in verschiedenen Ölen, welche bei der vorliegenden Erfindung bevorzugt eingesetzt werden.

**Tabelle 1**

| **Ölsorte** | **Ölsäure** (C 18:1) | **Linolsäure** (C 18:2) | **Linolensäure** (C 18:3) | **Eicosapentaensäure** (C 20:5) | **Docosaexaensäure** (C 22:6) |
|---|---|---|---|---|---|
| | Omega-9 | Omega-6 | Omega-3 | Omega-3 | Omega-3 |
| Olivenöl | 70 | 10 | 0 | 0 | 0 |
| Maisöl | 30 | 60 | 1 | 0 | 0 |
| Leinöl | 20 | 20 | 60 | 0 | 0 |
| Lebertran | 25 | 2 | 1 | 12 | 8 |
| Fischöl | 15 | 2 | 1 | 18 | 12 |

Die bei der erfindungsgemäßen Beschichtung eingesetzten Öle bzw. die Mischungen der Öle enthalten einen Anteil von mindestens 40 Gew.-% an ungesättigten Fettsäuren, bevorzugt einen Anteil von 50 Gew.-%, weiter bevorzugt einen Anteil von 60 Gew.-%, noch weiter bevorzugt einen Anteil von 70 Gew.-% und insbesondere bevorzugt einen Anteil von 75 Gew.-% an ungesättigten Fettsäuren. Sollten kommerziell erhältliche Öle, Fette oder Wachse verwendet werden, welche einen geringeren Anteil als 40 Gew.-% an Verbindungen mit mindestens einer Mehrfachbindung enthalten, so können ungesättigte Verbindungen in dem Maße zugesetzt werden, dass der Anteil an ungesättigten Verbindungen auf über 40 Gew.-% ansteigt. Bei einem Anteil von weniger als 40 Gew.-% sinkt die Polymerisationsgeschwindigkeit zu stark ab, so dass gleichmäßige Beschichtungen nicht mehr gewährleistet werden können.

Die Eigenschaft zur Polymerisation macht vor allem die Lipide mit hohen Anteilen mehrfach ungesättigter Fettsäuren zu hervorragenden Substanzen für die vorliegende Erfindung.

So besitzt die Linolsäure (Octadecadiensäure) zwei Doppelbindungen und die Linolensäure (Octadecatriensäure) drei Doppelbindungen. Eicosapentaensäure (EPA C20:5) hat fünf Doppelbindungen und Docosahexaensäure (DHA C22:6) besitzt sechs Doppelbindungen in einem Molekül. Mit der Anzahl der Doppelbindungen steigt auch die Bereitschaft zur Polymerisation.

Diese Eigenschaften der ungesättigten Fettsäuren und deren Mischungen sowie deren Neigung zur Autopolymerisation lässt sich zur biokompatiblen und flexiblen Beschichtung von Stents mit z.B. Fischöl, Lebertran oder Leinöl nutzen.

Linolsäure wird auch als cis-9, cis-12- Octadecadiensäure (chemische Nomenklatur) oder als Δ9,12-Octadecadiensäure oder als Octadecadiensäure (18:2) bzw. Octadecadiensäure 18:2 (n-6) bezeichnet (biochemische bzw. physiologische Nomenklatur). Bei Octadecadiensäure 18:2 (n-6) ist n die Anzahl an Kohlenstoffatomen und die Zahl "6" gibt die Position der letzten Doppelbindung an. 18:2 (n-6) ist somit eine Fettsäure mit 18 Kohlenstoffatomen, zwei Doppelbindungen und einem Abstand von 6 Kohlenstoffatomen von der letzten Doppelbindung bis zur äußeren Methylgruppe.

Bevorzugt werden für die vorliegende Erfindung die folgenden ungesättigten Fettsäuren als Substanzen eingesetzt, welche an der Polymerisationsreaktion teilnehmen bzw. Substanzen, welche diese Fettsäuren enthalten.

**Tabelle 1: Monoolefinische Fettsäuren**

| **Systematischer Name** | **Trivialname** | **Kurzform** |
|---|---|---|
| cis-9-Tetradecensäure | Myristoleinsäure | 14:1(n-5) |
| cis-9-Hexadecensäure | Palmitoleinsäure | 16:1(n-7) |
| cis-6-Octadecensäure | Petroselinsäure | 18:1(n-12) |
| cis-9-Octadecensäure | Ölsäure | 18:1(n-9) |
| cis-11-Octadecensäure | Vaccensäure | 18:1(n-7) |
| cis-9-Eicosensäure | Gadoleinsäure | 20:1(n-11) |
| cis-11-Eicosensäure | Gondoinsäure | 20:1(n-9) |
| cis-13-Docosensäure | Erucinsäure | 22:1(n-9) |
| cis-15-Tetracosensäure | Nervonsäure | 24:1(n-9) |
| t9-Octadecensäure | Elaidinsäure | |
| t11-Octadecensäure | t-Vaccensäure | |
| t3-Hexadecensäure | | trans-16:1 n-13 |

**Tabelle 2: Polyungesättigte Fettsäuren**

| **Systematischer Name** | **Trivialname** | **Kurzform** |
|---|---|---|
| 9,12-Octadecadiensäure | Linolsäure | 18:2(n-6) |
| 6,9,12-Octadecatriensäure | γ-Linolsäure | 18:3(n-6) |
| 8,11,14-Eicosatriensäure | Dihomo-γ-linolensäure | 20:3(n-6) |
| 5,8,11,14-Eicosatetraensäure | Arachidonsäure | 20:4(n-6) |
| 7,10,13,16-Docosatetraensäure | - | 22:4(n-6) |
| 4,7,10,13,16-Docosapentaensäure | - | 22:5(n-6) |
| 9,12,15-Octadecatriensäure | α-Linolensäure | 18:3(n-3) |
| 6,9,12,15-Octadecatetraensäure | Stearidonsäure | 18:4(n-3) |
| 8,11,14,17-Eicosatetraensäure | - | 20:4(n-3) |
| 5,8,11,14,17-Eicosapentaensäure | EPA | 20:5(n-3) |
| 7,10,13,16,19-Docosapentaensäure | DPA | 22:5(n-3) |
| 4,7,10,13,16,19-Docosahexaensäure | DHA | 22:6(n-3) |
| 5,8,11-Eicosatriensäure | Meadsäure | 20:3(n-9) |
| 9c 11t 13t Eleostearinsäure | | |
| 8t 10t 12c Calendinsäure | | |
| 9c 11t 13c Catalpicsäure | | |
| 4, 7, 9, 11, 13, 16, 19 Docosaheptadecansäure | Stellaheptaensäure | |
| | Taxolsäure | all-cis-5,9-18:2 |
| | Pinolensäure | all-cis-5,9,12-18:3 |
| | Sciadonsäure | all-cis-5,11,14-20:3 |

**Tabelle 3: Acetylenische Fettsäuren**

| **Systematischer Name** | **Trivialname** |
|---|---|
| 6-Octadecynoic acid | Taririnsäure |
| t11-Octadecen-9-ynoic ∼ | Santalbin- oder Ximeninsäure |
| 9-Octadecynoic ∼ | Stearolinsäure |
| 6-Octadecen-9-ynoic ∼ | 6,9-Octadeceninsäure∼ |
| t10-Heptadecen-8-ynoic ∼ | Pyrulinsäure |
| 9-Octadecen-12-ynoic ∼ | Crepenynsäure |
| t7,t11-Octadecadiene-9-ynoic ∼ | Heisterinsäure |
| t8,t10-Octadecadiene-12-ynoic ∼ | - |
| 5,8,11,14-Eicosatetraynoic ∼ | ETYA |

Nach der Durchführung der beschriebenen Polymerisation der einen linearen oder verzweigten und einen substituierten oder unsubstituierten Alkylrest mit mindestens einer Mehrfachbindung enthalten Substanzen, wobei die an der Polymerisationsreaktion teilnehmenden Substanzen einfach oder mehrfach ungesättigte Fettsäuren und/oder Mischungen aus diesen ungesättigten Fettsäuren in Form ihrer Triglyceride und/oder in nicht glyceringebundener, freier Form sind, wird eine Oberfläche eines Stents erhalten, welche zumindest teilweise mit einer polymeren Schicht versehen ist. Im Idealfall entsteht eine homogene gleichmäßig dicke polymere Schicht auf der gesamten äußeren Oberfläche des Stents, bzw. auf der gesamten mit Blut oder Blutprodukten in Kontakt tretenden Oberflächen des Stents.

Diese polymere Schicht auf der Oberfläche des Stents besteht aus den an der Polymerisationsreaktion teilnehmenden Substanzen und schließt die nicht aktiv an der Polymerisationsreaktion teilnehmenden Substanzen in der Polymermatrix ein. Vorzugsweise ist der Einschluss so ausgestaltet, dass die nicht an der Polymerisation teilnehmenden Substanzen, vor allem die Wirkstoffe aus der Polymermatrix hinausdiffundieren können.

Der biokompatible Überzug aus den polymerisierten Substanzen sorgt für die notwendige Blutverträglichkeit des Stent und stellt gleichzeitig einen geeigneten Träger für Wirkstoffe dar. Ein hinzugefügter Wirkstoff (oder Wirkstoffkombination), der über die Gesamtoberfläche des Stents gleichmäßig verteilt ist, bewirkt, dass der Bewuchs der Oberfläche mit Zellen, insbesondere glatten Muskel- und Endothelzellen, in einer kontrollierten Weise abläuft. Es findet somit keine rasche Besiedlung und Überwucherung einer Stentoberfläche mit Zellen statt, was zu einer Restenose führen könnte, wohingegen der Bewuchs der Stentoberfläche mit Zellen aber auch nicht durch eine hohe Medikamentenkonzentration vollkommen unterbunden wird, was die Gefahr einer Thrombose mit sich bringt.

Somit wird unter aktiver Unterstützung der Matrix gewährleistet, dass der Wirkstoff oder die Wirkstoffkombination, kovalent oder/und adhäsiv an die darunterliegende Schicht gebunden oder/und kovalent oder/und adhäsiv in die Schicht eingelagert, kontinuierlich und in geringen Dosen freigesetzt wird, so dass nicht die Besiedelung der Stentoberfläche mit Zellen unterbunden, jedoch eine Überbesiedelung verhindert wird. Diese Kombination beider Effekte verleiht der erfindungsgemäßen Oberfläche eines Stent die Fähigkeit, schnell in die Gefäßwand einzuwachsen und vermindert sowohl das Risiko einer Restenose als auch das Risiko einer Thrombose. Die Freisetzung des oder der Wirkstoffe erstreckt sich über einen Zeitraum von 1 bis 12 Monaten, bevorzugt über 1 - 2 Monate nach der Implantation.

Als Wirkstoffe werden antiproliferative Substanzen, antiphlogistische als auch antithrombotische, antimigrative und/oder antiangiogene Stoffe eingesetzt. Die Wirkstoffe werden einzeln oder kombiniert in gleicher oder unterschiedlicher Konzentration als nicht an der Polymerisationsreaktion teilnehmenden Substanzen eingesetzt. Diese Wirkstoffe können in Form einer ersten unteren Schicht auf die Oberfläche des Stents aufgetragen werden und die weiteren an der Polymerisation teilnehmenden Substanzen mit mindestens einem Alkylrest mit mindestens einer Mehrfachbindung sowie die anderen nicht an der Polymerisation teilnehmenden Substanzen können auf diese Wirkstoffschicht aufgetragen und dann polymerisiert werden, bevorzugt autopolymerisiert. Ferner besteht die Möglichkeit, die Wirkstoffe zu den an der Polymerisationsreaktion teilnehmenden Substanzen zu geben, so dass die Wirkstoffe in die polymere Matrix eingeschlossen werden. Durch einen derartiger Einschluss der Wirkstoffe wird einreicht, dass diese kontinuierlich aus der polymeren Matrix über die oben beschriebenen Zeiträume freigesetzt werden. Der Zeitraum der Wirkstofffreisetzung kann über den Polymerisationsgrad gesteuert werden. Je höher der Polymerisationsgrad, desto länger wird der Zeitraum, über den der oder die Wirkstoffe freigesetzt werden. Ferner besteht auch noch die Möglichkeit, den Wirkstoff oder die Wirkstoffkombination nach erfolgter Polymerisationsreaktion auf die polymere Matrix auf der Stentoberfläche aufzutragen oder nach Quellung der Polymermatrix den oder die Wirkstoffe in die Matrix einzubringen. Eine weitere Ausführungsform beinhaltet die kovalente Verknüpfung eines oder mehrerer Wirkstoffe mit der polymeren Matrix und/oder den Substanzen, welche nicht aktiv an der Polymerisationsreaktion teilgenommen haben. Es ist auch möglich, einen oder mehrere Wirkstoffe unter und/oder in und/oder auf der polymeren Matrix aufzubringen bzw. einzubringen, sei es vor, während oder nach der Polymerisationsreaktion.

Besonders bevorzugt sind Wirkstoffe, welche neben ihrer antiproliferativen Wirkung auch noch immunsuppressive Eigenschaften aufweisen und aus den Gruppen ausgewählt werden, welche Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, PI-88 (sulfatiertes Oligosaccharid), Melanocyte-stimulating hormon (α-MSH), aktiviertes Protein C, IL1-ß-Inhibitor, Thymosin α-1, Fumarsäure und deren Ester, Calcipotriol, Tacalcitol, Lapachol, ß-Lapachon, Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren wie Pentaerythrityltetranitrat und Syndnoeimine, S-Nitrosoderivate, Tamoxifen, Staurosporin, ß-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate wie 6-α-Hydroxy-Paclitaxel, Baccatin, Taxotere u.a., synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Tumstatin, Avastin, D-24851, SC-58125, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plasminogen-Aktivator Inhibitor-1, Plasminogen-Aktivator Inhibitor-2, Antisense Oligonucleotide, VEGF-Inhibitoren, IGF-1; Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, desulfatiertes und N-reacetyliertes Heparin, Gewebe-Plasminogen-Aktivator, GpIIb/IIIa-Plättchenmembranrezeptor, Faktor Xₐ-Inhibitor Antikörper, Heparin, Hirudin, r-Hirudin, PPACK, Protamin, Natriumsalz der 2-Methylthiazolidin-2,4-dicarbonsäure), Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon α, ß und γ, Histaminantagonisten, Serotoninblocker, Apoptoseinhibitoren, Apoptoseregulatoren wie p65, NF-kB oder Bci-xL-Antisense-Oligonukleotiden, Halofuginon, Nifedipin, Tocopherol, Vitamin B1, B2, B6 und B12, Folsäure, Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, D24851, SC-58125, Retinolsäure, Quinidin, Disopyrimid, Flecainid, Propafenon, Sotolol, Amidoron., natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Antimykotika wie Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A, B und C, Bruceantinoside C, Yadanzioside N und P, Isodeoxyelephantopin, Tomenphantopin A und B, Coronarin A, B, C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien-3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B und schwefelhaltige Aminosäuren wie Cystin sowie Salze und/oder Mischungen der vorgenannten Wirkstoffe umfassen.

Zudem bevorzugt ist eine Kombination von mehreren antiproliferativ wirkenden Substanzen oder von antiproliferativen Wirkstoffen mit immunsuppressiven Wirkstoffen. Bevorzugt für die vorliegende Erfindung sind Tacrolimus, Pimecrolimus, PI 88, Paclitaxel und seine Derivate, Trapidil, α- und ß-Estradiol, Natriumsalz der 2-Methylthiazolidin-2,4-dicarbonsäure), macrocyclisches Kohlensuboxid (MCS) und dessen Derivate, Sirolimus, Fumarsäure und ihre Ester, aktiviertes Protein C, Interleukin 1ß-Inhibitoren und Melanocyte-stimulating hormon (α-MSH), Cystin, Ellipticin, Bohemin, Indanocin, Colcemid und deren Derivate, Methionin sowie Salze und/oder Mischungen der vorgenannten Wirkstoffe.

Der Wirkstoff ist bevorzugt in einer pharmazeutisch aktiven Konzentration von 0,0001 - 10 mg pro cm² Oberfläche des Stent enthalten. Weitere Wirkstoffe können in ähnlicher Konzentration in derselben oder in weiteren Schichten enthalten sein. Bevorzugt beträgt die Konzentration an einem Wirkstoff auf der Oberfläche des Stents 0,001 - 5 mg pro cm² Oberfläche, weiter bevorzugt 0,005 - 3 mg pro cm² Oberfläche und insbesondere bevorzugt 0,01 - 2 mg pro cm² Oberfläche des Stents.

Stents mit einer erfindungsgemäß beschichteten Oberfläche lassen sich nach folgenden Verfahren herstellen:
a) Bereitstellen einer Oberfläche eines Stents, und
b) Aufbringen der Substanzen für die polymere Schicht, und
c) Polymerisation der mindestens einen Alkylrest mit mindestens einer Mehrfachbindung enthaltenden Substanzen durch Einwirkung von Wärme, Licht
und/oder Luftsauerstoff über diese Mehrfachbindung, wobei die Substanzen einfach oder mehrfach ungesättigte Fettsäuren und/oder Mischungen aus diesen ungesättigten Fettsäuren in Form ihrer Triglyceride und/oder in nicht glyceringebundener, freier Form sind.

Dabei werden die Substanzen für die polymere Schicht vorab gemischt und dann auf die Oberfläche des Stents aufgetragen. Zu den Substanzen für die polymere Schicht zählen die an der Polymerisationsreaktion teilnehmenden Substanzen, d.h. die aktiv an der Polymerisation teilnehmenden Substanzen, welche mindestens einen Alkylrest mit mindestens einer Mehrfachbindung enthalten, wobei diese Substanzen über die Polymerisation eben dieser mindestens einen Mehrfachbindung miteinander kovalent verknüpft werden. Ferner können die Substanzen für die polymere Schicht weitere, nicht aktiv an der Polymerisationsreaktion teilnehmende Substanzen enthalten. Diese nicht an der Polymerisation teilnehmende Substanzen umfassen beispielsweise die oben beschriebenen Wirkstoffe, Verbindungen, welche einen Alkylrest vergleichbar in der Kohlenstoffanzahl und den Substituenten mit dem Alkylrest der aktiv an der Polymerisation teilnehmenden Substanzen, jedoch mit dem Unterschied, dass der Alkylrest der nicht an der Polymerisation teilnehmenden Substanzen keine Mehrfachbindungen aufweist. Bei diesen Alkylresten handelt es sich vorzugsweise um gesättigte Fettsäurereste. Ferner gehören zu den nicht an der Polymerisationsreaktion teilnehmenden Substanzen gesättigte Fettsäuren, gesättigte Fettsäureester, gesättigte Fettsäurederivate, gesättigte Ether, gesättigte Lipide, Lipoide, gesättigte Fette und Öle, gesättigte Glyceride, gesättigte Triglyceride, gesättigte Glycolester, gesättigte Glycerinester, Wachse, biostabile oder bioabbaubare Polymere oder Mischungen der vorgenannten Substanzen.

Als Wachse eignen sich beispielsweise Bienenwachs, Carnaubawachs, Candelillawachs sowie Mischungen dieser Wachse.

Bevorzugt werden auch gesättigte Fettsäuren eingesetzt, welche bevorzugt einen Kettenlänge von mindestens 12 Kohlenstoffatomen aufweisen.

**Tabelle 4: gesättigte Fettsäuren**

| **Systematischer Name** | **Trivialname** | **Kurzform** |
|---|---|---|
| Dodecansäure | Laurinsäure | 12:0 |
| Tetradecansäure | Myristinsäure | 14:0 |
| Hexadecansäure | Palmitinsäure | 16:0 |
| Heptadecansäure | Margarinsäure | 17:0 |
| Octadecansäure | Stearinsäure | 18:0 |
| Eicosansäure | Arachinsäure | 20:0 |
| Docosansäure | Behensäure | 22:0 |
| Tetracosansäure | Lignocerinsäure | 24:0 |

Ferner sind auch Mischungen aus gesättigten Fettsäuren und/oder natürlichen Lipoiden wie Palmkernfett und Kokosnussfett bevorzugt.

Insbesondere geeignet sind sie folgenden biostabilen Polymere: Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Epoxyharze, ABS-Harze, EPDM-Gummis, Silicone wie Polysiloxane, Polyvinylhalogene und Copolymere, Celluloseether, Cellulosetriacetate, Chitosan und Copolymere und/oder Mischungen derselben.

Als bioabbaubare Polymere eignen sich beispielsweise Polyvalerolactone, Poly-ε-Decalactone, Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherester-multiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate, Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan] Polyhydroxypentan-säure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Heparansulfat, Heparin, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Modifikationen und Copolymere und/oder Mischungen der vorgenannten Substanzen

Diese Substanzen nehmen nicht an der Polymerisationsreaktion aktiv teil, d.h. sie werden nicht kovalent in der oder an die polymere Matrix gebunden, sondern in die polymere Matrix so eingebunden, dass sie ohne Spaltung einer kovalenten Bindung aus der Matrix herausgelöst werden könnten. Dies gilt insbesondere für die oben bezeichneten Wirkstoffe, welche in der Polymermatrix eingelagert sind und kontrolliert hinausdiffundieren.

Nach dem Auftragen der Substanzen für die polymere Schicht, also einer Mischung der an der Polymerisationsreaktion teilnehmenden und nicht teilnehmenden Substanzen wird die polymere Matrix erzeugt, indem die Substanzen, die mindestens einen Alkylrest mit mindestens einer Mehrfachbindung enthalten, durch Einwirkung von Wärme, Licht und/oder Luftsauerstoff über diese Mehrfachbindung polymerisiert werden. Bei dieser Polymerisation kann ein Katalysator in einer biokompatiblen, d.h. pharmakologisch verträglichen Konzentration eingesetzt werden. Als Katalysatoren kommen beispielsweise organische Radikale in Betracht oder organische Verbindungen, welche in Radikale zerfallen, wie Peroxide oder Diazoverbindungen. Ferner können auch anorganische Katalysatoren wie Kaliumpermanganat, Iod oder Brom eingesetzt werden.

Bei einem weiteren erfindungsgemäßen Verfahren wird vor dem Aufbringen der Substanzen für die polymere Schicht eine Schicht eines antiproliferativen, antiinflammatorischen und/oder antithrombotischen Wirkstoffs oder Wirkstoffgemisches aus den oben genannten Wirkstoffen aufgetragen. Auf diese Schicht werden dann die Substanzen für die polymere Schicht aufgetragen, welche ebenfalls einen oder mehrere der oben genannten Wirkstoffe enthalten können und dann polymerisiert.

Bevorzugt werden für die Polymerisationsreaktion solche Substanzen verwendet, welche autopolymerisieren.

Nach der Ausbildung der polymeren Schicht kann auf oder in diese eine weitere Wirkstoffschicht auf- oder eingebracht werden. Das Aufbringen kann adhäsiv oder auch kovalent erfolgen. Es ist nicht notwendig, einen Wirkstoff oder eine Wirkstoffkombination zu verwenden, welche bereits in einer unteren Schicht oder der polymeren Schicht enthalten ist. Ein nachträgliches Einbringen eines oder mehrerer Wirkstoffe in die polymere Schicht kann durch Quellung der polymeren Schicht und Eindiffundieren des oder der Wirkstoffe erfolgen.

Direkt auf die polymere Schicht oder bevorzugt auf diese äußere Wirkstoffschicht kann eine weitere zweite, dritte oder vierte Schicht aus einem biostabilen und/oder bioabbaubaren Polymeren aufgebracht werden. Diese äußere Schicht aus einem der oben genannten biostabilen oder bioabbaubaren Polymeren dient als Schutzschicht, welche eine kontrollierte Freisetzung der Wirkstoffe aus den darunterliegenden Schichten ermöglicht.

Anstelle dieser äußeren polymere Schicht kann auch eine weitere Schicht aus Substanzen für die darunterliegende Schicht gemäß den Reaktionsschritten b) und c) aufgebracht werden.

Die Substanzen für die polymere Schicht als auch für die optionale weitere polymere Schicht gemäß Reaktionsschritt b) und c) werden im Tauch- und/oder Sprühverfahren aufgebracht. Dabei wird der Wirkstoff oder die Wirkstoffmischung der nicht vollständig polymerisierten Sprüh- oder Tauchlösung bestehend aus den Substanzen für die polymere Schicht beigemischt.

Die oben genannten Wirkstoffe können adhäsiv und/oder kovalent an, in, auf und/oder unter einer Schicht gebunden sein.

Die vorliegende Erfindung betrifft somit Stents, deren Oberflächen nach einem der erfindungsgemäßen Verfahren beschichtet worden ist. Diese Stents sind bevorzugt für den direktren Kontakt mit Blut oder Blutprodukten geeignet.

Vorzugsweise weisen diese Stents nicht nur eine erfindungsgemäße hämokompatible Oberfläche auf, sondern enthalten zumindest einen der vorgenannten antiproliferativen, antiinflammatorischen und/oder antithrombotischen Wirkstoffe in einer pharmazeutisch aktiven Konzentration von 0,0001 - 10 mg pro cm² Stentoberfläche, bevorzugt 0,001 - 5 mg pro cm² Oberfläche, weiter bevorzugt 0,005 - 3 mg pro cm² Oberfläche und insbesondere bevorzugt 0,01 - 2 mg pro cm² Stentoberfläche.

Die den Stent unmittelbar bedeckende hämokompatible Schicht besteht bevorzugt aus einem polymeren Netzwerk mehrfach ungesättigter Fettsäuren. Diese Stents werden hergestellt, indem man herkömmliche in der Regel unbeschichtete Stents bereitstellt und bevorzugt adhäsiv eine biokompatible Schicht aufbringt, welche auf dem Stent zu einem flexiblen, dünnen den gesamten Stent gleichmäßig bedeckenden Film an der Luft und im Bedarfsfall unter Zugabe eines Katalysators in einer für den Menschen ungiftigen Konzentration polymerisiert. Wird ein Wirkstoff oder eine Wirkstoffkombination zugefügt, kann dies durch Beimischen in die Fettsäurelösung geschehen oder nachträglich durch Quellvorgänge in die bereits polymerisierte Matrix eindiffundieren oder vor der Beschichtung mit den Fettsäuren in einem separaten Arbeitsschritt aufgetragen werden.

Die herkömmlichen Stents, welche gemäß der erfindungsgemäßen Verfahren beschichtet werden können, bestehen aus Edelstahl, Nitinol oder anderen Metallen und Legierungen oder aus synthetischen Polymeren.
Eine weitere bevorzugte Ausführungsform der erfindungsgemäßen Stents weist eine Beschichtung auf, welche aus mindestens zwei Schichten besteht. Auch Multischichtsysteme finden Anwendung. Bei derartigen Mehrschichtsystemen wird als erste Schicht diejenige bezeichnet, welche direkt auf den Stent aufgebracht ist. Als zweite Schicht wird diejenige Schicht bezeichnet, welche auf die erste Schicht aufgebracht wird, usw.

Gemäß der Zweischichtausführung besteht die erste Schicht aus einer polymerisierten fettsäurehaltigen Schicht, welche im wesentlichen vollständig durch eine Schicht überzogen ist, die mindestens einen antiproliferativen, antiphlogistischen und/oder antithrombotischen Wirkstoff, kovalent oder/und adhäsiv gebunden, enthält. Ebenso werden auch Wirkstoffkombinationen eingesetzt, die sich in ihrer Wirkung gegenseitig unterstützen und ergänzen. Als polymerisierbare Öle werden pflanzliche und tierische Fetten mit hohen Anteilen ungesättigter Fettsäuren eingesetzt. Dazu zählen Leinöl, Hanföl, Maiskeimöl, Rapsöl, Sojaöl, Sonnenblumenöl, Weizenkeimöl, Distelöl, Traubenkernöl, Nachtkerzenöl, Schwarzkümmelöl, Algenöl, Fischöl, Lebertran und/oder Mischungen der vorgenannten Substanzen eingesetzt aber auch gezielt die diesen Mischungen zugrundeliegenden polymerisierbaren Fetten Linolensäure (ALA), Linolsäure, Eisosahexaensäure (EPA), Docosahexaensäure (DHA) als reine Substanzen oder in jedem Mischungsverhältnis. Die wirkstoffenthaltende Schicht bzw. Schichten wird langsam durch Blutbestandteile abgebaut, so dass der Wirkstoff entsprechend der Geschwindigkeit des Abbaus der Schicht freigesetzt wird oder sich entsprechend seines Elutionsverhalten aus der Matrix löst. Durch diesen biologischen Abbau und die dementsprechende Wirkstofffreisetzung wird ein Anwachsen von Zellen nur für eine gewisse Zeit stark reduziert und ein gezieltes kontrolliertes Anwachsen dort ermöglicht, wo die äußere Schicht bereits weitgehend abgebaut worden ist. Der biologische Abbau der polymeren Schicht erstreckt sich vorteilhafterweise über 1 bis 36 Monate, bevorzugt aber 1 - 6 Monate. In dieser Zeit spielen sich die wichtigen Heilungsprozesse ab.

Derartige Stents lassen sich durch ein Verfahren zur biokompatiblen, Beschichtung von Stents herstellen, dem folgendes Prinzip zugrunde liegt :
a. Bereitstellen eines unbeschichteten Stents
b. im wesentlichen vollständiges Überziehen der Oberfläche im Tauch- oder Sprühverfahren mit dem nicht polymerisierten Öl
   oder
b'. im wesentlichen vollständiges Überziehen oder/und unvollständiges Überziehen im Tauch- oder Sprühverfahren mit dem nicht polymerisierten Öl, welches mindestens einen Wirkstoff enthält.
c. Polymerisation der aufgebrachten Schicht an der Luft und bei Raumtemperatur oder erhöhter Temperatur.

Eine weitere Ausführung eines biokompatiblen Stents ist gegeben, wenn man das Öl auf die Oberfläche aufbringt und es nach beendeter Polymerisation und Aushärtung mit einem Wirkstoff oder einer Wirkstoffkombination durch Quellen in die Beschichtung eindiffundieren läßt. Des weiteren kann eine zweite reine Wirkstoffschicht auf die erste wirkstofffreie oder wirkstoffenthaltende Lipidschicht aufgetragen werden.
Für eine erfolgreiche gleichmäßige Beschichtung wird das Öl in einem leicht verdampfbaren organischen Lösungsmittel gelöst. Katalysatoren als auch synthetische Polymere, die verhindern sollen, das Öl von der Oberfläche abtropft, bevor es polymerisiert, können so leicht hinzugefügt werden.

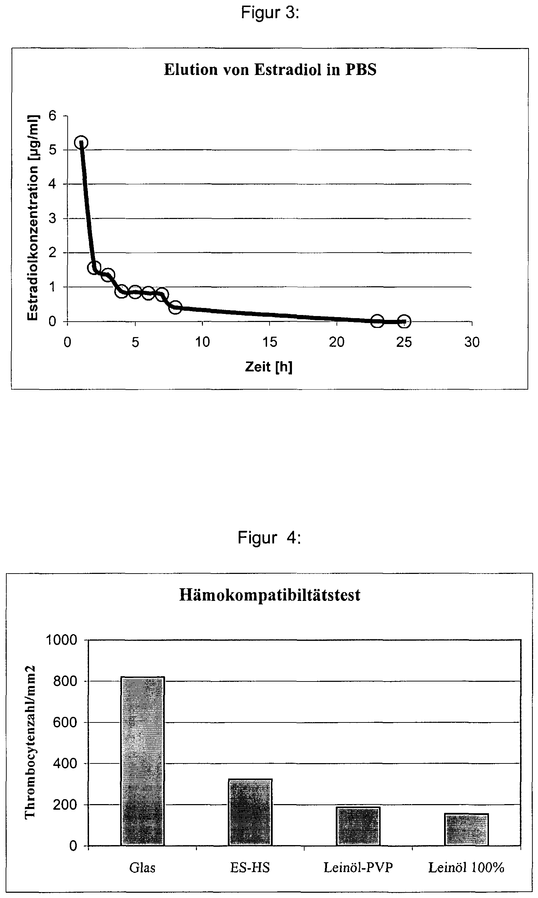

Die erfindungsgemäßen Stents lösen sowohl das Problem der akuten Thrombose (siehe Figur 4) als auch das Problem der Neointimahyperplasie nach einer Stentimplantation. Zudem eignen sich die erfindungsgemäßen Stents aufgrund ihrer Beschichtung, ob als Einzelschicht oder als Mehrschichtsystem, besonders gut zur kontinuierlichen Freisetzung eines oder mehrerer antiproliferativer, immunsuppressiver und/oder antithrombotische Wirkstoffe. Aufgrund dieser Fähigkeit der gezielten kontinuierlichen Wirkstofffreisetzung in erforderlicher Menge verhindern die erfindungsgemäß beschichteten Stents die Gefahr der Restenose.

### Beispiele

### Beispiel 1

Polymerisation von 100% igem Leinöl bei 80°C
Leinöl wird auf einem Objektträger als dünner Film aufgetragen und anschließend bei 80°C im Trockenschrank gelagert. Nach zwei Tagen ist die Polymerisation beendet. Man erhält eine gleichmäßige hellgelbe trockene, gut am Untergrund haftende polymere Schicht.

### Beispiel 2 :

Polymerisation von 100% igem Leinöl bei Raumtemperatur
Leinöl wird auf einem Objektträger als dünner Film aufgetragen und wird an der Luft und Einwirken von UV-Strahlung (Licht) gelagert. Nach 14 Tagen ist die Polymerisation beendet und das Öl ausgehärtet.

### Beispiel 3 :

Polymerisation von Mischungen aus Leinöl und Olivenöl (4:1)
Eine Mischung aus 80% Leinöl und 20% Olivenöl wird hergestellt und auf einem Objektträger als dünner Film aufgetragen und bei 80°C im Trockenschrank gelagert. Nach 2 Tagen ist das Öl zwar fest geworden, hat aber noch eine klebrige Oberfläche. Bei höheren Zusätzen von Olivenöl bleibt die flüssige Konsistenz erhalten.

### Beispiel 4:

Biokompatible Beschichtung von Stents mit Leinöl unter Zugabe eines Katalysators und einem synthetischen Polymeren, insbesondere Polyvinylpyrrolidon.

Nicht expandierte Stents aus medizinischem Edelstahl LVM 316 wurden im Ultraschallbad 15 Minuten mit Aceton und Ethanol entfettet und bei 100°C im Trockenschrank getrocknet. Anschließend wurden die Stents über Nacht mit demineralisiertem Wasser gewaschen.

Ca. 10mg KMnO₄ werden in 500µl Wasser gelöst und soviel PVP wie möglich zugegeben. Die Masse wird flächig auf einer Polypropylenunterlage ausgebreitet und bei Raumtemperatur über Nacht trocknen gelassen.
Von dieser spröden Masse werden 2,5mg in 1ml Chloroform gelöst und nach Zugabe von 10,5 µl Leinöl die resultierende Lösung mit einer Airbrush-Sprühpistole (EVOLUTION von Harder & Steenbeck) aus 6cm Entfernung auf einen rotierenden 18mm LVM Edelstahlstent gesprüht. Danach wurde der beschichtete Stent 24h bei 80°C gelagert.

### Beispiel 5:

Wirkstoffzugabe zu einem beschichteten Stent im Tauchverfahren

Der in Beispiel 5 beschichtete Stent wurde in einer Lösung von 600µg Paclitaxel in 1ml Ethanol getaucht und quellen gelassen. Nach Abschluß des Quellvorgangs wurde der Stent herausgenommen und getrocknet.

### Beispiel 6:

Biokompatible Beschichtung von Stents mit Leinöl und Paclitaxel
Nicht expandierte Stents aus medizinischem Edelstahl LVM 316 wurden im Ultraschallbad 15 Minuten mit Aceton und Ethanol entfettet und bei 100°C im Trockenschrank getrocknet. Anschließend wurden die Stents über Nacht mit demineralisiertem Wasser gewaschen.
Leinöl und Paclitaxel (80:20) wird im Mischungsverhältnis 1:1 in Chloroform gelöst und dann auf den gleichmäßig rotierenden Stent gesprüht. Nach Abdampfen des Chloroforms im leichten Luftstrom wird der Stent im Trockenschrank bei 80°C gelagert.

### Beispiel 7:

Biokompatible Beschichtung von Stents mit einer 0,25%igen ethanolischen Leinöl-Sprühlösung:
Nicht expandierte Stents aus medizinischem Edelstahl LVM 316 wurden im Ultraschallbad 15 Minuten mit Aceton und Ethanol entfettet und bei 100°C im Trockenschrank getrocknet. Anschließend wurden die Stents über Nacht mit demineralisiertem Wasser gewaschen.
Es wird eine 0,25Gew.% Sprühlösung aus Leinöl in Ethanol hergestellt und mit einer Sprühpistole gleichmäßig auf den um seine Achse rotierenden Stent gesprüht. Der beschichtete Stent wird im Trockenschrank bei 70°C über Nacht getrocknet.
Die durchschnittliche Coatingmasse beträgt 0,15mg ± 0,02mg.

### Beispiel 8:

Biokompatible Beschichtung von Stents mit einer ethanolischen Sprühlösung aus Leinöl und dem synthetischen Polymeren Polyvinylpyrrolidon (PVP)
Nach dem Reinigen der Stents wie in den Beispielen zuvor bereits beschrieben wird eine ethanolsche Sprühlösung hergestellt, die 0,25% Leinöl und 0,1% PVP enthält und mit einer Sprühpistole auf den um seine eigene Achse rotierenden Stent gleichmäßig aufgesprüht. Anschließend wird über Nacht bei 70°C getrocknet. Die Coatingmasse beträgt durchschnittlich 0,2mg ± 0,02mg.

### Beispiel 9:

Biokompatible Beschichtung von Stents mit Leinöl und dem synthetischen Polymeren Polyvinylpyrrolidon (PVP) im Zweischichtsystem unter Zugabe eines restenosehemmenden Wirkstoffes
Nach dem Reinigen der Stents wird eine erste Schicht aus 0,25Gew.-% Paclitaxel gelöst in Chloroform auf den Stent aufgesprüht. Nach dem Trocknen dieser Schicht bei Raumtemperatur wird die zweite Schicht einer Chloroform-Lösung mit 0,25% Leinöl und 0,1% PVP aufgesprüht. Nach Trocknung über Nacht bei 70°C wird die Coatingmasse mit 0,3mg ± 0,02mg bestimmt.

### Beispiel 10:

Biokompatible Beschichtung von Stents mit Leinöl und dem synthetischen Polymeren Polyvinylpyrrolidon (PVP) im Zweischichtsystem unter Zugabe eines restenosehemmenden Wirkstoffes
Nach dem Reinigen der Stents wird eine erste Schicht aus 0,25Gew.-% Leinöl wie Estradiol und 0,1% PVP gelöst in Ethanol auf den trockenen Stent aufgesprüht. Nach dem Trocknen dieser Schicht bei 70°C über Nacht wird die zweite Schicht einer Chloroform-Lösung mit 0,25% Leinöl und 0,1% PVP aufgesprüht. Nach dem Trocknen über Nacht bei 70°C wird die Coatingmasse mit 0,37mg ± 0,05mg bestimmt.

### Beispiel 11:

Biokompatible Beschichtung von Stents mit Leinöl und α-Linolensäure
Nach dem Reinigen der Stents mit Aceton und Ethanol, wie vorherig beschrieben, wird eine in Ethanol gelöste Mischung mit 0,20% Leinöl und 0,5% α-Linolensäure hergestellt und gleichmäßig auf den Stent aufgesprüht. Anschließend wird über Nacht bei 70°C getrocknet. Die Coatingmasse beträgt durchschnittlich 0,2mg ± 0,02mg.

### Figurenbeschreibung

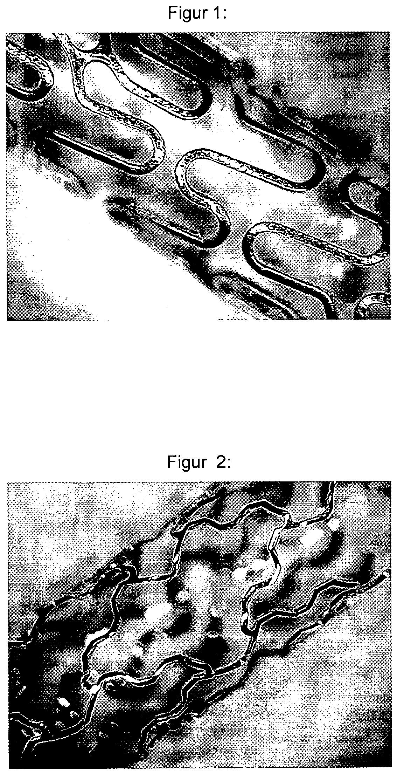

**Figur 1 und 2** zeigen lichtmikroskopische Aufnahmen von mit Leinöl-PVP (0,1%) beschichteten Stents. Die Beschichtung ist auch bei Designänderung gleichmäßig und zeigt keinerlei Verklebungen, die z.B. in den Bögen leicht entstehen kann.
**Figur 3** zeigt die Elutionsmessung von ß-Estradiol aus der Leinöl-PVP(0,1%)-Matrix.
**Figur 4** zeigt die Bestimmung der Thrombozytenadhäsion auf Glas, Endothelzellen-Heparansulfat (ESHS), Leinöl-PVP(0,1 %) und Leinöl (100%) in vitro. Die Messung erfolgte im dynamischen System der Baumgartnerkammer (modifiziert nach Sakarassien) mit humanem Vollblut.
Die Leinöl-Matrix mit und ohne PVP-Zusatz wird mit der bekannt stark thrombogenen Glasoberfläche und mit dem als antithrombogen klassifizierten Endothelzell-Heparansulfat verglichen. Das Diagramm macht deutlich, dass die Leinöl-Matrix mit und ohne PVP-Zusatz sich in diesem Vergleich mit Abstand als die Oberfläche auszeichnet, die die geringste Thrombocytenadhäsion bewirkt. Damit zeichnet sich das Leinöl als hämokompatible Matrix zur Beschichtung von Implantaten mit Blutkontakt aus. Es kristallisiert sich sogar eine weitere Verbesserung heraus, wenn auf den Zusatz von PVP verzichtet wird, da das als hämokompatibel erwiesene PVP im Mittel eine leichte Erhöhung der Thrombocytenadhäsion zeigt.

## Patentansprüche

1. Stent, dessen Oberfläche zumindest teilweise eine polymere Schicht umfasst, wobei die polymere Schicht aus mindestens 25 Gew.-% an einer Polymerisationsreaktion teilnehmenden Substanzen besteht welche einen mindestens eine Mehrfachbindung enthaltenden Alkylrest tragen, und wobei die
polymere Schicht durch Polymerisation der an der Polymerisationsreaktion teilnehmenden Substanzen nach der Auftragung auf die Stentoberfläche durch Einwirkung von Wärme, Licht und/oder Luftsauerstoff über diese Mehrfachbindung erhältlich ist, und die polymere Schicht Substanzen umfasst, wobei die an der Polymerisationsreaktion teilnehmenden Substanzen einfach oder mehrfach ungesättigte Fettsäuren und/oder Mischungen aus diesen ungesättigten Fettsäuren in Form ihrer Triglyceride und/oder in nicht glyceringebundener, freier Form sind.

2. Stent nach Anspruch 1, **dadurch gekennzeichnet, dass** die ungesättigten Fettsäuren aus der Gruppe ausgewählt werden umfassend Ölsäure, Eicosapentaensäure, Timnodonsäure, Docosahexaensäure, Arachidonsäure, Linolsäure, α-Linolensäure, γ-Linolensäure sowie Mischungen der vorgenannten Fettsäuren.

3. Stent nach einem der vorherigen Ansprüche, **dadurch gekennzeichnet**, das die nicht an der Polymerisationsreaktion teilnehmenden Substanzen gesättigte Fettsäuren, gesättigte Fettsäureester, gesättigte Fettsäurederivate, gesättigte Ether, gesättigte Lipide, Lipoide, gesättigte Fette und Öle, gesättigte Glyceride, gesättigte Triglyceride, gesättigte Glycolester, gesättigte Glycerinester, Wachse, biostabile oder bioabbaubare Polymere oder Mischungen der vorgenannten Substanzen umfassen.

4. Stent nach Anspruch 3, **dadurch gekennzeichnet**, das es sich bei den gesättigten Fettsäuren um die langkettigen Fettsäuren ab einer Kettenlänge von 12 C-Atomen sowie Mischungen derselben und/oder natürliche Lipoide wie Palmkernfett, Kokosnussfett sowie deren Mischungen handelt und dass es sich bei den Wachsen um Bienenwachs, Carnaubawachs, Candelillawachs und/oder deren Mischungen handelt.

5. Stent nach Anspruch 3, **dadurch gekennzeichnet, dass** die biostabilen Polymere aus der Gruppe ausgewählt werden umfassend Polyacrylsäure und Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Epoxyharze, ABS-Harze, Silicone, Polysiloxane, Polyvinylhalogene und Copolymere, Celluloseether, Cellulosetriacetate, Chitosan und Copolymere und/oder Mischungen derselben.

6. Stent nach Anspruch 3, **dadurch gekennzeichnet**, das die bioabbaubaren Polymere aus der Gruppe ausgewählt werden umfassend Polyvalerolactone, Poly-ε-Decalactone, Polylactide, Polyglycolide, Copolymere der Polylactide und Polyglycolide, Poly-ε-caprotacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polymaleinsäureanhydride, Polyhydroxymethacrylate, Polycyanoacrylate, Polycaprolactondimethylacrylate, Poly-b-Maleinsäure Polycaprolactonbutyl-acrylate, Multiblockpolymere aus Oligocaprolactondiole und Oligodioxanondiole, Polyetherester-multiblockpolymere, PEG, Poly(butylenterephtalat), Polypivotolactone, Polyglycolsäuretrimethyl-carbonate Polycaprolacton-glycolide, Poly(γ-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate, Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan] Polyhydroxypentansäure, Polyethylenoxidpropylenoxid, weiche Polyurethane, Polyurethane mit Aminosäurereste im Backbone, Polyetherester, Polyethylenoxid, Polyalkenoxalate, Polyorthoester sowie deren Copolymere, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, Hyaluronsäure, Heparansulfat, Heparin, Chondroitinsulfat, Dextran, β-Cyclodextrine, Copolymere mit PEG und Polypropylenglycol, Gummi arabicum, Guar, Gelatine, Collagen Collagen-N-Hydroxysuccinimid, Modifikationen und Copolymere und/oder Mischungen der vorgenannten Substanzen.

7. Stent nach einem der vorherigen Ansprüche, **dadurch gekennzeichnet**, das die nicht an der Polymerisationsreaktion teilnehmenden Substanzen antiproliferative, antiinflammatorische und/oder antithrombotische Wirkstoffe umfassen, ausgewählt aus der folgende Gruppe umfassend Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, PI-88 (sulfatiertes Oligosaccharid), Melanocyte-stimulating hormon (α-MSH), aktiviertes Protein C, IL1-ß-Inhibitor, Thymosin α-1, Fumarsäure und deren Ester, Calcipotriol, Tacalcitol, Lapachol, ß-Lapachon,Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren, Pentaerythrityltetranitrat, Syndnoeimine, S-Nitrosoderivate, Tamoxifen, Staurosporin, ß-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate, 6-α-Hydroxy-Paclitaxel, Baccatin, Taxotere, synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Tumstatin, Avastin, D-24851, SC-58125, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika, Cefadroxil, Cefazolin, Cefaclor, Cefotixin, Tobramycin, Gentamycin, Penicilline, Dicloxacillin, Oxacillin, Sulfonamide, Metronidazol, Antithrombotika, Argatroban, Aspirin, Abciximab, synthetisches Antithrombin, Bivalirudin, Coumadin, Enoxoparin, desulfatiertens und N-reacetyliertes Heparin, Gewebe-Plasminogen-Aktivator, GpIIb/IIIa-Plättchenmembranrezeptor, Faktor Xₐ-Inhibitor Antikörper, Heparin, Hirudin, r-Hirudin, PPACK, Protamin, Natriumsalz der 2-Methylthiazolidin-2,4-dicarbonsäure Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioproteaseinhibitoren, Prostacyclin, Vapiprost, Interferon α, ß und γ, Histaminantagonisten, Serotoninblocker, Apoptoseinhibitoren, Apoptoseregulatoren, p65, NF-kB oder Bcl-xL-Antisense-Oligonukleotiden, Halofuginon, Nifedipin, Tocopherol, Vitamin B1, B2, B6 und B12, Folsäure, Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron, natürliche und synthetisch hergestellte Steroide, Bryophyllin A, Inotodiol, Maquirosid A, Ghalakinosid, Mansonin, Streblosid, Hydrocortison, Betamethason, Dexamethason, nichtsteroidale Substanzen (NSAIDS), Fenoporfen, Ibuprofen, Indomethacin, Naproxen, Phenylbutazon, antivirale Agentien, Acyclovir, Ganciclovir, Zidovudin, Antimykotika, Clotrimazol, Flucytosin, Griseofulvin, Ketoconazol, Miconazol, Nystatin, Terbinafin, antiprozoale Agentien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A, B und C, Bruceantinoside C, Yadanzioside N und P, Isodeoxyelephantopin, Tomenphantopin A und B, Coronarin A, B, C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Daphnoretin, Lariciresinol, Methoxylariciresinol, Syringaresinol, Umbelliferon, Afromoson, Acetylvismion B, Desacetylvismion A, Vismion A und B, schwefelhaltige Aminosäuren, Cystin sowie Salze und/oder Mischungen der vorgenannten Wirkstoffe.

8. Stent nach einem der vorherigen Ansprüche, wobei unter und/oder in und/oder auf der polymeren Schicht mindestens ein antiproliferativer, antiinflammatorischer und/oder antithrombotischer Wirkstoff gemäß Anspruch 7 kovalent und/oder adhäsiv gebunden ist.

9. Stent nach Anspruch 7 oder 8, **dadurch gekennzeichnet, dass** der antiproliferative, antiinflammatorische und/oder antithrombotische Wirkstoff gemäß Anspruch 10 in einer pharmazeutisch aktiven Konzentration von 0,001 - 10 mg pro cm² Oberfläche des Stents enthalten ist.

10. Verfahren zur biokompatiblen Beschichtung von Stents umfassend die Schritte:
a) Bereitstellen einer Oberfläche eines Stents, und
b) Aufbringen der mindestens einen Alkylrest mit mindestens einer Mehrfachbindung enthaltenden Substanzen für die polymere Schicht, und
c) Polymerisation der Substanzen über diese Mehrfachbindung durch Einwirkung von Wärme, Licht und/oder Luftsauerstoff, wobei die Substanzen einfach oder mehrfach ungesättigte Fettsäuren und/oder Mischungen aus diesen ungesättigten Fettsäuren in Form ihrer Triglyceride und/oder in nicht glyceringebundener, freier Form sind.

11. Verfahren zur biokompatiblen Beschichtung von Stents umfassend die Schritte:
a) Bereitstellen einer Oberfläche eines Stents, und
a') Aufbringung einer Schicht eines antiproliferativen, antiinflammatorischen und/oder antithrombotischen Wirkstoffs oder Wirkstoffgemisches gemäß Anspruch 7, und
b) Aufbringen der mindestens einen Alkylrest mit mindestens einer Mehrfachbindung enthaltenden Substanzen für die polymere Schicht, und
c) Polymerisation der Substanzen über diese Mehrfachbindung durch Einwirkung von Wärme, Licht und/oder Luftsauerstoff, wobei die Substanzen einfach oder mehrfach ungesättigte Fettsäuren und/oder Mischungen aus diesen ungesättigten Fettsäuren in Form ihrer Triglyceride und/oder in nicht glyceringebundener, freier Form sind.

12. Verfahren gemäß Anspruch 10 oder 11 des weiteren umfassend den Schritt d) d) Aufbringung und/oder Einbringung einer Schicht eines antiproliferativen, antiinflammatorischen und/oder antithrombotischen Wirkstoffs oder Wirkstoffgemisches gemäß Anspruch 7 auf und/oder in die polymere Schicht.

13. Verfahren nach einem der Ansprüche 10 - 12, **dadurch gekennzeichnet, dass** der antiproliferative, antiinflammatorische und/oder antithrombotische Wirkstoff gemäß Anspruch 7 kovalent und/oder adhäsiv in und/oder an die jeweilige Schicht gebunden ist.

14. Verfahren nach einem der Ansprüche 10 - 13, **dadurch gekennzeichnet, dass** der antiproliferative, antiinflammatorische und/oder antithrombotische Wirkstoff oder die Wirkstoffkombination gemäß Anspruch 7 in einer pharmazeutisch aktiven Konzentration von 0,001 - 10 mg pro cm² Oberfläche des Stents vorhanden ist.

15. Stent erhältlich nach einem der Verfahren gemäß eines der Ansprüche 10 - 14.

## Claims

1. A stent the surface of which comprises at least partially a polymer layer, wherein the polymer layer consists of at least 25% by weight of substances participating in the polymerization reaction which bear an alkyl moiety containing at least one multiple bond and wherein the polymer layer is obtainable via this multiple bond by polymerization of the substances participating in the polymerization reaction after deposition onto the stent surface by means of exposure to heat, light and/or aerial oxygen, and the polymer layer comprises substances, wherein the substances participating in the polymerization reaction are mono- or polyunsaturated fatty acids and/or mixtures of these unsaturated fatty acids in form of their triglycerides and/or in glycerin unbound, free form.

2. The stent according to claim 1, **characterized in that** the unsaturated fatty acids are selected from the group comprising: oleic acid, eicosapentaenoic acid, timnodonic acid, docosahexaenoic acid, arachidonic acid, linoleic acid, α-linolenic acid, γ-linolenic acid as well as mixtures of the aforementioned fatty acids.

3. The stent according to any one of the preceding claims, **characterized in that** the substances not participating in the polymerization reaction comprise saturated fatty acids, saturated fatty acid esters, saturated fatty acid derivatives, saturated ethers, saturated lipids, lipoids, saturated fats and oils, saturated glycerides, saturated triglycerides, saturated glycol esters, saturated glycerin esters, waxes, biostable or biodegradable polymers or mixtures of the aforementioned substances.

4. The stent according to claim 3, **characterized in that** in the case of the saturated fatty acids the long-chain fatty acids beyond a chain length of 12 carbon atoms as well as mixtures thereof and/or natural lipoids, palm kernel fat, coconut fat as well as their mixtures are concerned and that in the case of the waxes beeswax, carnauba wax, candelilla wax and/or mixtures thereof are concerned.

5. The stent according to claim 3, **characterized in that** the biostable polymers are chosen from the group comprising polyacrylic acid and polyacrylates, polymethylmethacrylate, polybutylmethacrylate, polyacrylamide, polyacrylonitriles, polyamides, polyetheramides, polyethylenamine, polyimides, polycarbonates, polycarbourethanes, polyvinylketones, polyvinylhalogenides, polyvinylidenhalogenides, polyvinylethers, polyvinylaromates, polyvinylesters,
polyvinylpyrollidones, polyoxymethylenes, polyethylene, polypropylene, polytetrafluoroethylene, polyurethanes, polyolefine elastomeres, polyisobutylenes, EPDM gums, fluorosilicones, carboxymethylchitosanes, polyethyleneterephthalate, polyvalerates, carboxymethylcellulose, cellulose, rayon, rayontriacetates, cellulosenitrates, celluloseacetates, hydroxyethylcellulose, cellulosebutyrates, celluloseacetatebutyrates, ethylvinylacetate copolymers, polysulphones, epoxy resins, ABS resins, EPDM gums, silicones, polysiloxanes, polyvinylhalogenes and copolymers, celluloseethers, cellulosetriacetates, chitosanes and copolymers and/or mixtures of these substances.

6. The stent according to claim 3, **characterized in that** the biodegradable polymers are chosen from the group comprising polyvalerolactones, poly-ε-decalactones, polylactides, polyglycolides, copolymers of the polylactides and polyglycolides, poly-ε-caprolactone, polyhydroxybutanoic acid, polyhydroxybutyrates, polyhydroxyvalerates, polyhydroxybutyrate-co-valerates, poly(1,4-dioxane-2,3-diones), poly(1,3-dioxane-2-one), poly-para-dioxanones, polyanhydrides, polymaleic anhydrides, polyhydroxymethacrylates, fibrin, polycyanoacrylates, polycaprolactonedimethylacrylates, poly-b-maleic acid, polycaprolactonebutylacrylates, multiblock polymers from oligocaprolactonedioles and oligodioxanonedioles, polyetherester multiblock polymers PEG and poly(butyleneterephtalates), polypivotolactones, polyglycolic acid trimethylcarbonates, polycaprolactone-glycolides, poly(g-ethylglutamate), poly(DTH-iminocarbonate), poly(DTE-co-DT-carbonate), poly(bisphenol-A-iminocarbonate), polyorthoesters, polyglycolic acid trimethyl-carbonates, polytrimethylcarbonates, polyiminocarbonates, poly(N-vinyl)-pyrrolidone, polyvinylalcoholes, polyesteramides, glycolated polyesters, polyphosphoesters, polyphosphazenes, poly[p-carboxyphenoxy)propane], polyhydroxypentanoic acid, polyanhydrides, polyethyleneoxide-propyleneoxide, soft polyurethanes, polyurethanes with amino acid moieties in the backbone, polyetheresters, polyethyleneoxide, polyalkeneoxalates, polyorthoesters as well as their copolymers, carrageenans, fibrinogen, starch, collagen, protein based polymers, polyamino acids, synthetic polyamino acids, zein, modified zein, polyhydroxyalkanoates, pectic acid, actinic acid, modified and non modified fibrin and casein, carboxymethylsulphate, albumin, moreover hyaluronic acid, heparansulphate, heparin, chondroitinesulphate, dextran, b-cyclodextrines and copolymers with PEG and polypropyleneglycol, gummi arabicum, guar, gelatin, collagen, collagen-N-Hydroxysuccinimide, modifications and copolymers and/or mixtures of the aforementioned substances.

7. The stent according to any one of the preceding claims, **characterized in that** the substances not participating in the polymerization reaction comprise antiproliferative, antiinflammatoric and/or antithrombotic active agents chosen from the group comprising sirolimus (rapamycin), everolimus, pimecrolimus, somatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoposide, teniposide, nimustine, carmustine, lomustine, cyclophosphamide, 4-hydroxycyclophosphamide, estramustine, melphalan, ifosfamide, trofosfamide, chlorambucil, bendamustine, dacarbazine, busulfan, procarbazine, treosulfan, temozolomide, thiotepa, daunorubicin, doxorubicin, aclarubicin, epirubicin, mitoxantrone, idarubicin, bleomycin, mitomycin, dactinomycin, methotrexate, fludarabine, fludarabine-5'-dihydrogenphosphate, cladribine, mercaptopurine, thioguanine, cytarabine, fluorouracil, gemcitabine, capecitabine, docetaxel, carboplatin, cisplatin, oxaliplatin, amsacrine, irinotecan, topotecan, hydroxycarbamide, miltefosine, pentostatin, aldesleukin, tretinoin, asparaginase, pegaspargase, anastrozole, exemestane, letrozole, formestane, aminoglutethimide, adriamycin, azithromycin, spiramycin, cepharantin, smc proliferation inhibitor-2w, epothilone A and B, mitoxantrone, azathioprine, mycophenolatmofetil, c-myc-antisense, b-myc-antisense, betulinic acid, camptothecin, PI-88 (sulfated oligosaccharide), melanocyte stimulating hormone (α-MSH), activated protein C, IL-1β inhibitor, thymosine α-1, fumaric acid and its esters, calcipotriol, tacalcitol, lapachol, β-lapachone, podophyllotoxin, betulin, podophyllic acid 2-ethylhydrazide, molgramostim (rhuGM-CSF), peginterferon α-2b, lenograstim (r-HuG-CSF), filgrastim, macrogol, dacarbazine, basiliximab, daclizumab, selectin (cytokine antagonist), CETP inhibitor, cadherines, cytokinin inhibitors, COX-2 inhibitor, NFkB, angiopeptin, ciprofloxacin, camptothecin, fluroblastin, monoclonal antibodies, which inhibit the muscle cell proliferation, bFGF antagonists, probucol, prostaglandins, 1,11-dimethoxycanthin-6-one, 1-hydroxy-11-methoxycanthin-6-one, scopoletin, colchicine, NO donors, pentaerythritol tetranitrate and syndnoeimines, S-nitrosoderivatives, tamoxifen, staurosporine, β-estradiol, α-estradiol, estriol, estrone, ethinylestradiol, fosfestrol, medroxyprogesterone, estradiol cypionates, estradiol benzoates, tranilast, kamebakaurin and other terpenoids, which are applied in the therapy of cancer, verapamil, tyrosine kinase inhibitors (tyrphostines), cyclosporine A, paclitaxel and derivatives thereof, 6-α-hydroxy-paclitaxel, baccatin, taxotere, synthetically produced as well as from native sources obtained macrocyclic oligomers of carbon suboxide (MCS) and derivatives thereof, mofebutazone, acemetacin, diclofenac, lonazolac, dapsone, o-carbamoylphenoxyacetic acid, lidocaine, ketoprofen, mefenamic acid, piroxicam, meloxicam, chloroquine phosphate, penicillamine, tumstatin, avastin, D-24851, SC-58125, hydroxychloroquine, auranofin, sodium aurothiomalate, oxaceprol, celecoxib, β-sitosterin, ademetionine, myrtecaine, polidocanol, nonivamide, levomenthol, benzocaine, aescin, ellipticine, D-24851 (Calbiochem), colcemid, cytochalasin A-E, indanocine, nocodazole, S 100 protein, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor-1, plasminogen activator inhibitor-2, antisense oligonucleotides, VEGF inhibitors, IGF-1; active agents from the group of the antibiotics, cefadroxil, cefazolin, cefaclor, cefotaxim, tobramycin, gentamycin, penicillins, dicloxacillin, oxacillin, sulfonamides, metronidazol, antithrombotics, argatroban, aspirin, abciximab, synthetic antithrombin, bivalirudin, coumadin, enoxaparin, desulphated and N-reacetylated heparin, tissue plasminogen activator, GpIIb/IIIa platelet membrane receptor, factor Xₐ inhibitor antibodies, heparin, hirudin, r-hirudin, PPACK, protamin, sodium salt of 2-methylthiazolidine-2,4-dicarboxylic acid, prourokinase, streptokinase, warfarin, urokinase, vasodilators, dipyramidole, trapidil, nitroprussides, PDGF antagonists, triazolopyrimidine and seramin, ACE inhibitors, captopril, cilazapril, lisinopril, enalapril, losartan, thio-protease inhibitors, prostacyclin, vapiprost, α, β and γ interferon, histamine antagonists, serotonin blockers, apoptosis inhibitors, apoptosis regulators, p65, NF-kB or Bcl-xL antisense oligonucleotides, halofuginone, nifedipine, tocopherol, vitamin B1, B2, B6 and B12, folic acid, tranilast, molsidomine, tea polyphenols, epicatechin gallate, epigallocatechin gallate, Boswellinic acids and derivatives thereof, leflunomide, anakinra, etanercept, sulfasalazine, etoposide, dicloxacillin, tetracycline, triamcinolone, mutamycin, procainamid, D24851, SC-58125, retinoic acid, quinidine, disopyramide, flecainide, propafenone, sotalol, amidorone, natural and synthetically produced steroids, bryophyllin A, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone and other antiviral agents such as acyclovir, ganciclovir and zidovudine, antimycotics, clotrimazole, flucytosine, griseofulvin, ketoconazole, miconazole, nystatin, terbinafine, antiprozoal agents, chloroquine, mefloquine, quinine, moreover natural terpenoids, hippocaesculin, barringtogenol-C21-angelate, 14-dehydroagrostistachin, agroskerin, agrostistachin, 17-hydroxyagrostistachin,
ovatodiolids, 4,7-oxycycloanisomelic acid, baccharinoids B1, B2, B3 and B7, tubeimoside, bruceanol A, B and C, bruceantinoside C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-a-senecioyloxychaparrin, taxamairin A and B, regenilol, triptolide, moreover cymarin, apocymarin, aristolochic acid, anopterin, hydroxyanopterin, anemonin, protoanemonin, berberine, cheliburin chloride, cictoxin, sinococuline, bombrestatin A and B, cudraisoflavone A, curcumin, dihydronitidine, nitidine chloride, 12-β-hydroxypregnadiene-3,20-dione, bilobol, ginkgol, ginkgolic acid, helenalin, indicine, indicine-N-oxide, lasiocarpine, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetine, pancratistatin, liriodenine, oxoushinsunine, aristolactam-AII, bisparthenolidine, periplocoside A, ghalakinoside, ursolic acid, deoxypsorospermin, psychorubin, ricin A, sanguinarine, manwu wheat acid, methylsorbifolin, sphatheliachromen, stizophyllin, mansonine, strebloside, akagerine, dihydrousambarensine, hydroxyusambarine, strychnopentamine, strychnophylline, usambarine, usambarensine, berberine, liriodenine, oxoushinsunine, daphnoretin, lariciresinol, methoxylariciresinol, syringaresinol, umbelliferon, afromoson, acetylvismione B, desacetylvismione A, vismione A and B and sulfur containing amino acids, cystine as well as salts and/or mixtures of the aforementioned active agents.

8. The stent according to any one of the preceding claims, wherein at least one antiproliferative, antiinflammatoric and/or antithrombotic active agent according to claim 7 is bound covalently and/or adhesively under and/or in and/or on the polymer layer.

9. The stent according to claim 7 or 8, **characterized in that** the antiproliferative, antiinflammatoric and/or antithrombotic active agent according to claim 7 is contained in a pharmaceutically active concentration of 0.001 to 10 mg per cm² surface of the stent.

10. A method for the biocompatible coating of stents comprising the steps:
a) providing a surface of a stent,
and
b) deposition of the substances containing at least one alkyl moiety with at least one multiple bond for the polymer layer,
and
c) polymerization of the substances containing at least one alkyl moiety with at least one multiple bond via this multiple bond by means of exposure to heat, light and/or aerial oxygen, wherein the substances are mono- or polyunsaturated fatty acids and/or mixtures of these unsaturated fatty acids in form of their triglycerides and/or in glycerin unbound, free form.

11. A method for the biocompatible coating of stents comprising the steps:
a) providing a surface of a stent,
and
a') deposition of layer of an antiproliferative, antiinflammatoric and/or antithrombotic active agent or active agent mixture according to claim 7, and
b) deposition of the substances containing at least one alkyl moiety with at least one multiple bond for the polymer layer,
and
c) polymerization of the substances containing at least one alkyl moiety with at least one multiple bond via this multiple bond by means of exposure to heat, light and/or aerial oxygen, wherein the substances are mono- or polyunsaturated fatty acids and/or mixtures of these unsaturated fatty acids in form of their triglycerides and/or in glycerin unbound, free form.

12. The method according to claim 10 or 11 further comprising the step d):
d) deposition and/or incorporation of a layer of an antiproliferative, antiinflammatoric and/or antithrombotic active agent or active agent mixture according to claim 7 on and/or in the polymer layer.

13. The method according to any one of claims 10 to 12, **characterized in that** the antiproliferative, antiinflammatoric and/or antithrombotic active agent according to claim 7 is bound covalently and/or adhesively in and/or to the respective layer.

14. The method according to any one of claims 10 to 13, **characterized in that** the antiproliferative, antiinflammatoric and/or antithrombotic active agent or the active agent combination according to claim 7 is present in a pharmaceutically active concentration of 0.001 to 10 mg per cm² surface of the stent.

15. The stent in accordance with the method of any one of claims 10 to 14.

## Revendications

1. Un stent dont la surface comprend au moins partiellement une couche polymérique, ladite couche polymérique étant constituée par au moins 25% en poids substances participant à la réaction de polymérisation et présentant au moins un résidu alkyle contenant au moins une liaison multiple, et
dans lequel la couche polymérique est obtenue à travers cette liaison multiple par polymérisation des substances participant à la réaction de polymérisation sous l'action de la chaleur, de la lumière et/ou de l'oxygène atmosphérique suite à leur application sur la surface du stent,
et la couche polymérique comprend des substances, où les substances participant à la réaction de polymérisation sont des acides gras mono ou poly insaturés et/ou des mélanges de ces acides gras insaturés sous forme de triglycéride et/ou sous forme libre non liée à la glycérine.

2. Le stent selon la revendication 1, **caractérisé en ce que**, les acides gras insaturés sont sélectionnés à partir du groupe comprenant: l'acide oléique, l'acide eicosapentaénoïque, l'acide timnodonique, l'acide docosahexaénoïque, l'acide arachidonique, l'acide linoléique, l'acide α-linolénique, l'acide γ-linolénique, ainsi que des mélanges des acides gras mentionnés ci-dessus.

3. Le stent selon l'une quelconque des revendications précédentes, **caractérisé en ce que**, les substances ne participant pas à la réaction de polymérisation comprennent des d'acides gras saturés, des esters d'acides gras saturés, des dérivés d'acides gras saturés, des éthers saturés, des lipides saturés, des lipoïdes, des graisses saturées et des huiles saturées, des glycérides saturés, des triglycérides saturés, des esters de glycol saturés, des esters de glycérol saturés, des cires, des polymères biostables ou biodégradables ou des mélanges des substances précitées.

4. Le stent selon la revendication 3, **caractérisé en ce que** les acides gras saturés sont des acides gras à longue chaine ayant une longueur de chaine supérieure à 12 atomes de carbone, ainsi que des mélanges de ceux-ci et/ou des lipoïdes naturels, comme la graisse de noyau de palme, la graisse de noix de coco, ainsi que des mélanges de celles-ci et les cires sont la cire d'abeille, la cire de carnauba, la cire de candelilla, et/ou des mélanges de celles-ci.

5. Le stent selon la revendication 3, **caractérisé en ce que**, les polymères biostables sont sélectionnés à partir du groupe comprenant: acide polyacrylique et polyacrylates, polyméthylméthacrylate, polybutylméthacrylate, polyacrylamide, polyacrylonitriles, polyamides, polyétheramides, polyéthylène amine, polyimides, polycarbonates, polycarbouréthanes, polyvinyle cétones, polyvinyle halogénés, polyvinylidène halogénés, polyvinyle éthers, polyvinyle aromates, polyvinyle esters, polyvinyle pyrollidones, polyoxyméthylènes, polyéthylène, polypropylène, polytétrafluoroéthylène, polyuréthanes, élastomères de polyoléfine, polyisobutylènes, gommes EPDM, fluorosilicones, carboxyméthyle chitosanes, polytéréphtalate d'éthylène, polyvalérates, carboxyméthyle cellulose, carboxyméthyle cellulose, cellulose, rayon, rayontriacétates, nitrates de cellulose, acétates de cellulose, hydroxyéthyl cellulose, butyrates de cellulose, cellulose acétate butyrate, copolymères d'acétate de vinyle et d'éthyle, polysulfones, résines époxy, résines ABS, silicone, polysiloxane, polyvinyle halogénés et copolymères, éthers de cellulose, triacétate de cellulose, chitosan et copolymères et/ou mélanges de ceux-ci.

6. Le stent selon la revendication 3, **caractérisé en ce que**, les polymères biodégradables sont sélectionnés à partir du groupe comprenant :
polyvalérolactones, poly-ε-décalactones, polylactides, polyglycolides, copolymères polylactides/polyglycolides, poly-ε caprolactone, polyhydroxybutyrates, polyhydroxyvalérates, poly(acide hydroxybutyrique), polyhydroxybutyrate-co-valérates, poly(1,4-dioxane-2,3-diones), poly(1,3-dioxane-2-ones), poly-para-dioxanones, polyanhydrides, poly(anhydride maléique), poly-hydroxy-méthacrylates, poly-cyano-acrylates, polycaprolactone diméthylacrylates, acide poly-b-maléique, polycaprolacton-butyl-acrylates, polymères à blocs multiples d'oligocaprolactondiols et d'oligodioxanondiols, polyéther-ester-polymères à blocs multiples, polyéthylène glycol, polybutylène téréphthalate, polypivotolactones, acide polyglycolique triméthyl carbonates, poly(γ-éthyl-glutamate), poly(DTH-iminocarbonate), poly(DTE-co-DT-carbonate), poly(bisphénol A-iminocarbonate), polyorthoesters, acide polyglycolique triméthyl carbonates, polytriméthyl carbonates, polyiminocarbonates, poly(N-vinyl)-pyrrolidone, polyvinyl alcohols, polyester amides, polyesters glycolés, polyphosphoesters, polyphosphazènes, poly[p-carboxyphénoxy)propane], acide polyhydroxy-péntanoique, polyéthylène oxide propylène oxide, polyuréthanes mous, polyuréthanes ayant des résidus amino acide dans le squelette, polyétheresters, polyéthylène oxide, polyalkènoxalates, polyorthoesters ainsi que copolymères de ceux-ci, carraghénane, fibrinogène, starch, collagène, polymères à base de protéine, poly(amino acides), poly(amino acides) synthétiques, zéine, zéine modifiée, polyhydroxyalkanoates, acide pectinique, acide actinique, caséine et fibrine modifiée et non-modifiée, carboxyméthyl sulfate, albumine, acide hyaluronique, héparansulfate, héparines, chondroitin sulfate, dextrane, β-cyclodextrines, copolymères avec PEG et polypropylène glycol, gomme arabique, guar, gélatine, collagène, collagène N-hydroxysuccinimide, modifications et copolymères et/ou des mélanges des substances précitées.

7. Le stent selon une quelconque des revendications précédentes, **caractérisé en ce que**, les substances non participant à la réaction de polymérisation comprennent des agents actifs antiprolifératives, anti-inflammatoires et/ou antithrombotiques sélectionnés à partir du groupe comprenant: sirolimus (rapamycine), everolimus, pimecrolimus, samostatine, tacrolimus, roxythromycine, dunaimycine, ascomycine, bafilomycine, érythromycine, midécamycine, josamycine, concanamycine, clarithromycine, troleadomycine, folimycine, cérivastatine, simvastatine, lovastatine, fluvastatine, rosuvastatine, atorvastatine, pravastatine, pitavsatatine, vinblastine, vincristine, vindesine, vinorelbine, étoboside, teniposide, nimustibe, carmustine, lomustine, cyclophosphamide, 4-hydroxyoxycyclo phosphamide, éstramustine, melphalan, ifosfamide, trofosfamide, cloroambucil, bendamustine, dacarbazine, bussulfan, procarbazine, tréosulfan, trémozolomide, thiotépa, daunorubicine, doxorubicine, aclarubicine, épirubicine, mitoxantrone, idarubicine, bléomycine, mitomycine, dactinomycine, méthotrexate, fludarabine, fludarabine-5'-dihydrogéne phosphate, cladribine, mercaptopurine, thioguanine, cytarabine, fluorouracile, gemcitabine, capecitabin, docétaxel, carboplatine, cisplatine, oxaliplatine, amsacrine, irinotécan, topotécan, hydroxycarbamide, miltéfosine, pentostatine, aldesleukine, trétinoine, asparaginase, pegasparase, anastrozole, exémestane, létrozole, formestane, aminoglutéthimide, adriamycinq, azithromycine, spiramycine, cepharantine, inhibiteur de la prolifération de cellules des muscles lisses-2w, épothilone A et B, mitoxanthrone, azathioprine, mycophénolate mofétil, c-myc antisense, b-myc antisense, acide bétulinique, camptothécine, oligosaccharides sulfatés PI-88, hormone mélanotrope (α-MSH), protéine C activée, inhibiteur de l'IL1-ß, thymosine α-1, acid fumarique et ses esters, calcipotriol, tacalcitol, lapachol, ß-lapachon, podophyllotoxine, bétuline, acide podophyllique-2-éthylhydrazide, molgramostim (rhuGM-CSF), pegintérferon α-2b, lenograstim (r-HuG-CSF), filgrastim, macrogol, dacarbazine, basiliximab, daclizumab, sélectine (antagoniste de cytokine), inhibiteurs de la CETP, cadhèrine, inhibiteurs des cytokines, inhibiteur de la COX-2, NFkB, angiopeptine, ciprofloxacine, camptothécine, fluroblastin, anticorps monoclonaux qui inhibitent la prolifération des cellules musculaires, antagonistes du bFGF, probucol, prostaglandine, 1,11-diméthoxycanthin-6-one, 1-hydroxy-11-méthoxycanthin-6-one, scopolectine, colchicine, donneurs de NO, pentaérythrityl tetranitrate, sydnoimine, dérivés S-nitroso, tamoxifène, staurosporine, β-estradiol, α-estradiol, estriol, estrone, éthinyl estradiol, fosfestrol, médroxyprogestérone, cypionates d'estradiol, benzoates d'estradiol, tranilast, kamebakaurine et autres terpénoides, utilisés dans la thérapie du cancer, verapamil, inhibiteurs de la tyrosine kinase (tyrphostins), cyclosporine A, paclitaxel et ses dérivés, 6-α-hydroxy-paclitaxel, baccatin, taxotères, oligomères macrocycliques de suboxyde du carbone (MCS) obtenus synthétiquement ou à partir des sources naturelles et leur dérivés, mofebutazone, acemétacine, diclofenac, lonazolac, dapsone, acide o-carbamoyl-phenoxyacétique, lidocaine, kétoprofène, acide méfénamique, piroxicam, méloxicam, chloroquine phosphate, pénicillamine, tumstatine, avastine, D-24851, SC-58125, hydroxychloroquine, auranofine, aurothiomalate de sodium, oxacéprol, célécoxib, β-sitostérine, ademétionine, myrtécaine, polidocanol, nonivamide, lévomenthol, benzocaine, aescine, ellipticine, D-24851 (Calbiochem), colcémide, cytochalasines A-E, indanocine, nocadazole, protéine S 100, bacitracine, antagonistes du récepteur de la vitronectine, azélastine, stimulateur de la guanidyl cyclase, inhibiteur tissulaire des métalloprotéinases 1 et 2, acides nucléiques libres, acides nucléiques incorporés dans des virus transmetteurs, fragments de l'ADN et l'ARN, inhibiteur-1 de l'activateur du plasminogène, inhibiteur-2 de l'activateur du plasminogène, les oligonucléotides antisens, les inhibiteurs du VEGF, l'IGF-1, agents actifs du groupe des antibiotiques, céfadroxil, céfazolin, céfaclor, céfoxitin, tobramycine, gentamicine, pénicilline, dicloxacilline, oxacilline, sulfonamides, métronidazole, antithrombotiques, argatroban, aspirine, abciximab, antithrombine synthétique, nivalirudine, coumadin, énoxoparine, héparines desulfatées et N-ré-acétylés, activateur tissulaire du plasminogène, recepteur membranaire de la plaquette GpIIb/IIIa, anticorps de l'inhibiteur du facteur Xa, héparine, hirudine, r-hirudine, PPACK, protamine, sel de sodium de l'acide 2-méthylthiazolidin-2-4 dicarboxylique, prourokinase, streptokinase, warfarine, urokinase, vasodilatateurs,
dipyramidole, trapidil, nitroprussides, antagonistes du PDGF, triazolopyrimidine, seramin, inhibiteurs de l'ACE, captopril, cilazapril, lisinopril, énalapril, losartan, inhibiteurs de la thioprotéase, prostacycline, vapiprost, interféron α, β et γ, antagonistes d'histamine, bloqueurs de la sérotonine, inhibiteurs de l'apoptose, régulateurs de l'apoptose, p65, NF-kB ou des oligonucléotides antisense Bcl-xL, halofuginone, nifédipine, tocophérol, vitamine B1, B2, B6 et B12, acide folique, tranirast, molsidomine, polyphénols du thé, gallate d'épicatéchine, gallate d'épigallocatéchine, acides boswelliques et leur dérivés, léflunomide, anakinra, étanercept, sulfasalazine, etoposid, dicloxacylline, tétracycline, triamcinolone, mutamycine, procainimide, acide rétinoïque, quinidine, disopyrimide, flécaïnide, propafénone, sotolol, amidorone, stéroïdes naturels et synthétiques, bryophylline A, inotodiol, maquiroside A, ghalakinoside, mansonine, strebloside, hydrocortisone, bétaméthasone, déxaméthasone, substances non-stéroïdiennes (NSAIDS), fénoprofène, ibuprofène, indomèthacine, naproxen, phénylbutazone, agents antiviraux, acyclovir, ganciclovir, zidovudine, antimycotiques, clotrimazole, flucytosine,, griséofulvine, kétoconazole, miconazole, nystatin, terbinafine, agents antiprotozoaires, chloroquine, méfloquine, quinine, terpénoides naturels, hippocaesculine, barringtogénol-C21-angelate, 14-déhydroagrostistachine, agroskerine, agrostistachine, 17-hydroxyagrostistachine, ovatodiolids, acide 4,7-oxycycloanisomelique, baccharinoids B1, B2, B3 et B7, tubeimoside, brucéanol A, B et C, bruceantinoside C, yadanziosides N et P, isodéoxyelephantopine, tomenphantopine A et B, coronarine A, B, C et D, acide ursolique, acide hyptatique A, zeorin, iso-iridogermanal, maytenfoliol, effusantine A, excisanine A et B, longikaurine B, sculponeatin C, kamebaunin, leukamenin A et B, 13,18-déhydro-6-alpha-senecioyloxychaparrin, taxamairin A et B, regenilol, triptolide, cymarin, apocymarin, acide aristolochique, anopterine, hydroxyanopterine, anémonin, protoanémonin, berbérine, chlorure de cheliburin, cictoxine, sinococuline, bombrestatin A et B, cudraisoflavone A, curcumine, dihydronitidine, chlorure de nitidine, 12-β-hydroxypregnadièn-3,20-dione, bilobol, ginkgol, acide ginkgolique, hélénaline, indicine, indicine-N-oxyde, lasiocarpine, inotodiol, glycoside 1a, podophyllotoxine, justicidine A et B, larréatin, malloterin, mallotochromanol, isobutyrylmallotochromanol, maquiroside A, marchantine A, maytansine, lycoridicine, margétine, pancratistatin, liriodènine, oxoushinsunine, aristolactame-AII, bisparthenolidine, periplocoside A, acide ursolique, déoxypsorospermine, psychorubine, ricin A, sanguinarine, acide du blé manwu, méthylsorbifoline, chromones de spathelia, stizophylline, mansonine, strebloside, akagerine, dihydro usambaraensine, hydroxyusambarine, strychnopentamine, strychnophylline, usambarine, usambarensine, berbérine, liriodenine, daphnoretine, laricirésinol, méthoxylaricirésinol, syringarésinol, umbellifèrone, afromosone, acétylvismione B, desacétylvismione A, vismione A et B, acides aminés contenant du souffrem cystine, ainsi que des sels et/ou des mélanges des substances précitées.

8. Le stent selon l'une quelconque des revendications précédentes, dans lequel au moins un agent antiprolifératif, anti-inflammatoire et/ou anti-thrombotique selon la revendication 7 est lié de manière covalente et/ou adhésive en dessous de et/ou dans et/ou au-dessus de la couche polymérique.

9. Le stent selon revendication 7 ou 8, **caractérisé en ce que**, l'agent antiprolifératif, anti-inflammatoire et/ou anti-thrombotique selon la revendication 7 a une concentration pharmaceutiquement active de 0,001 - 10 mg per cm² de surface de stent.

10. Un procédé pour le revêtement biocompatible d'un stent, comprenant les étapes suivantes :
a) fournir une surface d'un stent,
et
b) appliquer des substances contenant au moins un résidu alkyle avec au moins une liaison multiple pour la couche polymérique,
et
c) polymérisation des substances contenant au moins un résidu alkyle avec au moins une liaison multiple, à travers la liaison multiple, sous l'action de la chaleur, de la lumière et/ou de l'oxygène atmosphérique,
où les substances sont des acides gras mono ou poly insaturés et/ou des mélanges de ces acides gras insaturés sous forme de triglycéride et/ou sous forme libre non liée à la glycérine.

11. Un procédé pour le revêtement biocompatible d'un stent, comprenant les étapes suivantes :
a) fournir une surface d'un stent,
et
a') appliquer une couche d'un agent antiprolifératif, anti-inflammatoire et/ou anti-thrombotique ou d'un mélange d'agents actives selon la revendication 7,
et
b) appliquer des substances contenant au moins un résidu alkyle avec au moins une liaison multiple pour la couche polymérique,
et
c) polymérisation des substances contenant au moins un résidu alkyle avec au moins une liaison multiple, à travers la liaison multiple, sous l'action de la chaleur, de la lumière et/ou de l'oxygène atmosphérique,
où les substances sont des acides gras mono ou poly insaturés et/ou des mélanges de ces acides gras insaturés sous forme de triglycéride et/ou sous forme libre non liée à la glycérine.

12. Le procédé selon revendication 10 ou 12, comprenant de plus l'étape d) d) appliquer et/ou incorporer une couche d'un agent antiprolifératif, anti-inflammatoire et/ou anti-thrombotique ou d'un mélange d'agents actives selon la revendication 7 au-dessus et/ou dans la couche polymérique.

13. Le procédé selon une quelconque des revendications 10 - 12, **caractérisé en ce que**, l'agent antiprolifératif, anti-inflammatoire et/ou anti-thrombotique selon la revendication 7 est lié de manière covalente et/ou adhésive dans et/ou à la couche respective.

14. Le procédé selon une quelconque des revendications 10 - 13, **caractérisé en ce que** l'agent antiprolifératif, anti-inflammatoire et/ou anti-thrombotique ou le mélange d'agents actives selon la revendication 7 est présent à une concentration pharmaceutiquement active de 0,001 - 10 mg per cm² de surface de stent.

15. Un stent obtenu par le procédé selon une quelconque des revendications 10 - 14.
